(19) Europäisches Patentamt
European Patent Office
Office européen des brevets

(11) Veröffentlichungsnummer: **0 116 358**
**B1**

(12) # EUROPÄISCHE PATENTSCHRIFT

(45) Veröffentlichungstag der Patentschrift: **27.04.88**

(51) Int. Cl.⁴: **A 61 K 9/18,** A 61 K 31/557

(21) Anmeldenummer: **84101169.5**

(22) Anmeldetag: **06.02.84**

(54) **Komplexe von Prostaglandinen.**

(30) Priorität: **12.02.83 DE 3304867**
**12.02.83 DE 3304880**
**12.02.83 DE 3304864**

(43) Veröffentlichungstag der Anmeldung:
**22.08.84 Patentblatt 84/34**

(45) Bekanntmachung des Hinweises auf die Patenterteilung:
**27.04.88 Patentblatt 88/17**

(84) Benannte Vertragsstaaten:
**AT BE CH DE FR GB IT LI LU NL SE**

(56) Entgegenhaltungen:
**EP-A-0 066 475**
**FR-A-2 447 191**
**US-A-3 826 823**

**JOURNAL OF PHARMACEUTICAL SCIENCES, Band 67, Nr. 7, Juli 1978, Seiten 971-975, Washington D.C.,USA, HO-LEUNG FUNG et al.: "Molecular interaction between E-prostaglandins and selected polymers and its potential utilization in oral dosage form design"**
**PATENTS ABSTRACTS OF JAPAN, Band 3, Nr. 7(C-34), 24. Januar 1979, Seite 79C34**
**JOURNAL OF PHARMACEUTICAL SCIENCES, Band 69, Nr. 11, November 1980, Seiten 1271-1273,A.S. HARRIS et al.: "Preparation, characterization and stability of new prostaglandin E2 gel for local administration"**
**JOURNAL OF PHARMACEUTICAL SCIENCES, Band 70, Nr. 7, Juli 1981, Seiten 738-743, Washington D.C.,USA, K.H. FRÖMMING et al.: "Sorption properties of cross-linked insoluble polyvinylpyrrolidone"**

(73) Patentinhaber: **BAYER AG, Konzernverwaltung RP Patentabteilung, D-5090 Leverkusen 1 Bayerwerk (DE)**

(72) Erfinder: **Streuff, Bernhard, Dr., Morgengraben 10, D-5000 Köln 80 (DE)**
Erfinder: **Mathes, Detlef, Dr., Robert- Lütters- Weg 5, D-5600 Wuppertal 12 (DE)**
Erfinder: **Schade, Bernd, Dr., Morgengraben 6, D-5000 Köln 80 (DE)**
Erfinder: **Schorsch, Ulrich, Dr., Lindenbecker Weg 64, D-4000 Düsseldorf 12 (DE)**

(56) Entgegenhaltungen: (Fortsetzung)

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 116 358 B1

LIBER, STOCKHOLM 1988

**Beschreibung**

Die vorliegende Erfindung betrifft Komplexe von Prostaglandinen mit Crosspovidone, vorverkleisterter Stärke oder Dextranen ein Verfahren zur Herstellung dieser Adsorbate, ihre Verwendung in oder als Arzneimittel und Arzneimittel bzw. pharmazeutische Formulierungen enthaltend Komplexe von Prostaglandinen an Crosspovidone, vorverkleisterter Stärke oder Dextranen.

Prostaglandine sind Naturstoffe, die über den Weg des Arachidonsäure-Stoffwechsels gebildet werden und sehr intensive und spezifische Wirkungen haben. Als natürliche Prostaglandine sind z. B. bekannt Prostaglandine der Typen:

A

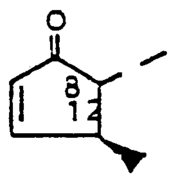

$(A_1, A_2)$

B

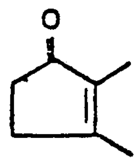

$(B_1, B_2)$

E

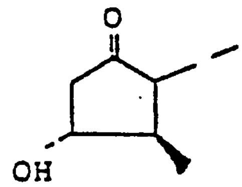

$(E_1, E_2, E_3)$

F

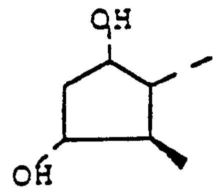

$(F_{1\alpha}, F_{2\alpha})$

Die einzelnen natürlichen Verbindungen und deren synthetische Analoga unterscheiden sich durch unterschiedliche Substitutionen in den beiden Seitenketten an C-8 und C-12 des jeweiligen Fünfringes, z. B. durch OH-Gruppen, Doppelbindungen, aliphatische Substituenten, Carbonsäurereste und deren Ester.

Wegen ihrer intensiven und spezifischen biologischen und pharmakologischen Aktivitäten erlangen die Prostaglandine zunehmendes Interesse. Diejenigen des E-Typs im weiteren PGE genannt - sind sehr gut zur Behandlung von Hypertension, Asthma bronchiale, Magen-Darm-Ulcera und Thrombosen sowie zur Einleitung von Wehen und Aborten bei Säugern, also Menschen und Tieren, geeignet.

Es ist jedoch bekannt, daß Prostaglandine, insbesondere Prostaglandin E-Derivate, relativ instabil sind. Diese Instabilität hat sich bisher als Hindernis für eine breite pharmazeutische Anwendung der PGE-Verbindungen erwiesen. Die Zersetzung der E-Derivate verläuft pH-abhängig unter Eliminierung von Wasser zu den entsprechenden PGA- bzw. PGB-Derivaten:

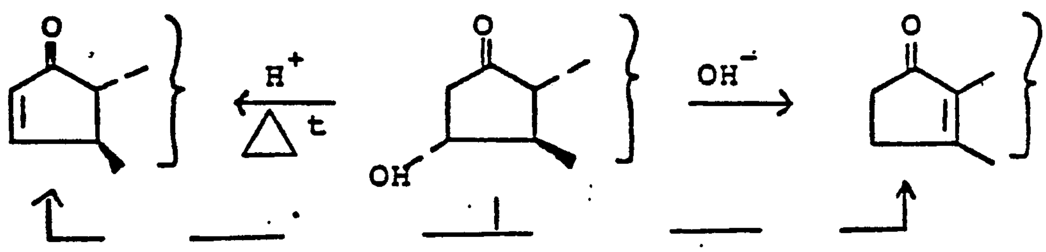


PGA PGE PGB

Es hat daher nicht an Versuchen gefehlt, diese labilen Prostaglandin-Derivate zu stabilisieren.

Aus Eur. J. Phamacol. 4, 1968, S. 416-420 ist es bekannt, daß Lösungen von Prostaglandinen in Methanol in Abhängigkeit vom pH-Wert im Falle von $PGE_1$ und $PGE_2$ bis zu 40 Tagen bei Raumtemperatur stabil sind. Eine medizinische Verwendung ist jedoch schon wegen der Toxizität des Methanols ausgeschlossen.

Nach einer Mitteilung in Lipids 8, 10, 1973, S. 592-594 verliert $PGE_2$ in Ethanol bei einer Aufbewahrungstemperatur von 4°C in einem Monat 5-12 % der Aktivität und zersetzt sich in Kochsalzlösung so rasch, daß nach 15 Tagen nur noch 58-82 % der ursprünglichen Aktivität vorhanden sind.

Überraschenderweise wurde nun gefunden, daß durch Adsorption an Crosspovidone, vorverkleisterte Stärke oder Dextrane die Stabilität von Prostaglandinen wesentlich verbessert werden kann, so daß z. B. der breite Einsatz als Arzneimittel möglich ist.

Aus Journal of Pharmaceutical Sciences, Band 67, Nr. 7, July 1978, S. 971-975 ist eine molekulare Interaktion zwischen Prostaglandinen des E-Typs mit ausgewählten Polymeren bekannt. Sinn der in dieser Veröffentlichung beschriebenen Aufgabe war die Beantwortung der Frage, ob es möglich ist, die Lösungsrate der prodrugs zu erhöhen. Dies geschieht durch Manipulation in den Seitenketten von Prostaglandinen des E-Typs. Es wurde gefunden, daß höher schmelzende prodrugs im Prinzip stabiler sind.

Gegenstand der vorliegenden Verbindung sind daher an vorverkleisterte Stärke oder Dextrane gebundene Prostaglandine des E-Typs, in denen das C16-Atom substituiert ist sowie Komplexe von Crosspovidonen,

vorverkleisterter Stärke oder Dextranen und speziellen Prostaglandinen, die nachfolgend mit I bis XVI bezeichnet sind.

Ein weiterer Gegenstand der vorliegenden Erfindung ist ein Verfahren zur Herstellung stabiler Prostaglandinformulierungen, bei dem das Prostaglandin des E-Typs, bei dem das C16-Atom substituiert ist, in gelöster Form auf Crosspovidone, vorverkleisterte Stärke oder Dextrane aufgebracht und anschliessend getrocknet wird.

Ferner betrifft die vorliegende Erfindung die Verwendung der stabilen Prostaglandinformulierungen zur Herstellung von Arzneimitteln sowie Formulierungen enthaltend die stabilisierten Prostaglandin-Komplexe. Die Erfindung betrifft daher auch Formulierungen, enthaltend an vorverkleisterte Stärke oder Dextran gebundene Prostaglandine des E-Typs, in denen das C16-Atom substituiert ist sowie feste Arzneiformen enthaltend an Crosspovidone, vorverkleisterte Stärke oder Dextran gebundene Prostaglandine der Gruppe I bis XVI.

Unter Crosspovidone im Sinne der vorliegenden Erfindung werden durch weitere Polymerisation quervernetztes Polyvinylpyrrolidon verstanden, insbesondere solches, das in Wasser unlöslich ist. Bevorzugt werden solche Crosspovidone unter die vorliegende Erfindung fallend angesehen, die die Spezifikation von NF XV erfüllen (National Formulary, 15 Edition, Official November 1, 1981, the United States Pharmacopoeial Convention, Inc).

Unter vorverkleisterter Stärke im Sinne der vorliegenden Erfindung wird natürliche Stärke jedweder Herkunft, die durch einen physikalischen oder chemischen Prozeß verkleistert und anschließend getrocknet worden ist, verstanden. Bevorzugt wird unter vorverkleisterter Stärke eine solche verstanden, welche die Spezifizierung von NF XV erfüllt. NF XV ist veröffentlicht als "The National Formulary", fifteenth edition, Official from July 1, 1980, United States Pharmacopoeial Convention, Inc.

Unter Dextranen im Sinne der vorliegenden Erfindung werden aus Glukoseresten in 1,4- und 1,6-Bindung aufgebaute, kettenförmige, verzweigte Polysaccharide verstanden. Bevorzugt werden unter Dextranen solche Polysaccharide verstanden, die im wesentlichen aus durch 1,6-glukosidische Bindungen charakterisierte Anhydro-D-glucopyranose-Einheiten bestehen, die bei vollständiger Hydrolyse D-Glucose liefern; bevorzugt weisen diese Polysaccharide ein Molekulargewicht zwischen ca. 1000 bis ca. 20.000.000 auf, besonders bevorzugt zwischen ca. 10.000 bis ca. 1.000.000.

Besonders bevorzugt im Sinne der vorliegenden Erfindung sind Komplexe von Prostaglandinen an Crosspovidone, deren Herstellung und Verwendung.

16-Methyl-1-11 α, 16 RS-Trihydroxyprost- 13 E-en-9on der nachstehenden Formel I

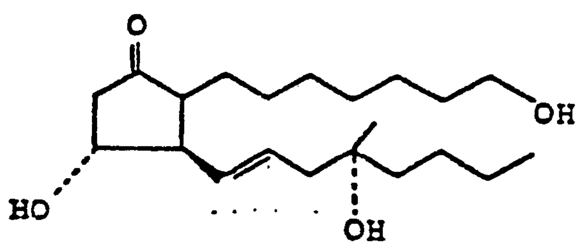

(I)

wird als ganz besonders bevorzugtes Prostaglandin im Sinne der vorliegenden Erfindung verstanden.

Da die erfindungsgemäß einzusetzenden Prostaglandine pharmazeutisch extrem stark wirkende Substanzen darstellen und die Dosierungen unterhalb des Milligramm-Bereiches liegen können, ist das erfindungsgemäße Verfahren besonders gut geeignet, die Wirkstoffe in einfacher Weise auf große Mengen Trägerstoff aufzuziehen. Bei sehr niedrigen Dosierungen ist eine große Menge Trägerstoff sogar wegen der einheitlichen Verteilung des Wirkstoffs pro Dosiseinheit (content uniformity) wünschenswert.

Bei den fertigen, nach der Trocknung erhaltenen erfindungsgemäßen Komplexen von Prostaglandinen an Crosspovidone, vorverkleisterte Stärke oder Dextrane soll das Gewichtsverhältnis der Prostaglandine zu Crosspovidone, vorverkleisterte Stärke oder Dextrane zwischen 1 : 1 bis 1 : 10 000 liegen, bevorzugt zwischen 1: 10 und 1 : 1000 und ganz besonders bevorzugt zwischen 1 : 100 bis 1 : 500.

Bei dem erfindungsgemäßen Verfahren werden stabile Prostaglandin-Formulierungen erzielt, bei denen auch die vorstehend angegebenen Gewichtsverhältnisse bevorzugt sind. Bei dem erfindungsgemäßen Verfahren wird das entsprechende Prostaglandin mit dem Crosspovidone, vorverkleinerte Stärke oder Dextran innig vermischt und von diesem gebunden.

Dies kann z. B. dadurch geschehen, daß das Prostaglandin, in einem organischen oder wäßrigen Lösungsmittel gelöst und Crosspovidone, vorverkleisterte Stärke oder Dextran gleichfalls in dieser Lösung entsprechend der oben angegebenen Gewichtsverhältnisse suspendiert wird.

Aus dieser Suspension wird der Komplex durch schonendes Trocknen erhalten. Als erfindungsgemäße Trocknung kann z. B. eine Gefriertrocknung, Vakuumtrocknung, Vakuumwalzentrocknung, Wirbelschichttrocknung oder Sprühtrocknung durchgeführt werden. Diese Aufzählung ist jedoch nicht abschließend, sondern es können auch andere schonende Trocknungsverfahren angewendet werden.

Eine andere Verfahrensvariante besteht darin, die gelösten Prostaglandine auf Crosspovidone, vorverkleisterte Stärke oder Dextran direkt aufzutragen. Dies kann z. B. durch Aufsprühen der Wirkstofflösung

auf das Trägermaterial durch Tauchen, Berieseln oder Wirbelschichtauftragung geschehen. Die Trocknung kann dann wieder nach einer der vorstehend erwähnten Methoden erfolgen.

Das Verfahren mit anschließender Trocknung wird so durchgeführt, daß die erhaltenen Komplexe einen bestimmten Restwassergehalt besitzen. Erfindungsgemäß und nach dem erfindungsgemäßen Verfahren hergestellte Komplexe sollen einen Restwassergehalt, bezogen auf das Gesamtgewicht, zwischen 0,1 bis 15 Gew.-%, vorzugsweise zwischen 0,5 und 10 Gew.-% und ganz besonders bevorzugt zwischen 1 und 5 Gew.-% aufweisen. Es ist erfindungsgemäß nicht erwünscht, einen Restwassergehalt unter 0,1 Gew.-% zu erreichen. Die Restwassergehalte beziehen sich auf fertige Komplexe, die nach der Restimmungsmethode von Karl Fischer (Europäisches Arzneibuch, Bd.I, 1974, S. 107) analysiert werden.

Im allgemeinen weisen die Komplexe nach ihrer Trocknung einen Prostaglandingehalt von 0,01-50 Gew.-%, vorzugsweise von 0,1-10 Gew.-%, besonders bevorzugt von 0,2-1 Gew.-% auf.

Überraschenderweise ist bei den erfindungsgemäßen Adsorbaten die Abbaugeschwindigkeit der Prostaglandine so weit reduziert, daß diese bei einer Lagertemperatur von etwa 30°C über einen Zeitraum von mehr als einem Jahr deutlich weniger als 10 % Gehaltsabfall aufweisen. Aufgrund der vorliegenden Untersuchungsergebnisse sind die erfindungsgemäßen Komplexe über mehr als 2 Jahre stabil und weisen einen Gehaltsabfall an Wirkstoff unter 8 % auf.

Die Komplexe können alleine oder unter Zusatz von geeigneten Hilfsstoffen zu festen oder halbfesten Arzneiformen wie auch zu Sprays oder Inhalaten verarbeitet werden. Als Hilfsstoffe kommen z. B. Stärke, Cellulose, Zucker, Mannit, Polyvinylpyrrolidon, Talkum, Stearinsäure und deren Salze, langkettige Aliphaten, wie z. B. hydriertes Ricinus- oder Baumwollsaatöl, Wachse, Fette und flüssige, halbfeste und feste Kohlenwasserstoffe sowie Polyethylenglykole, Emulgatoren und andere pharmazeutische Hilfsstoffe, in Frage.

Die Herstellung fester Arzneiformen wie Tabletten, Kapseln und Pulver, kann mittels einer Direkttablettierung bzw. Direktabfüllung der reinen Komplexe oder auch unter Zusatz weiterer Hilfsstoffe wie auch über die Zwischenstufe eines Granulations- und/oder Mischvorganges erfolgen. Als Hilfsstoffe zur Herstellung von Tabletten, Kapseln oder Pulver werden bevorzugt Stärke, Cellulose, Zucker, Mannit, Polyvinylpyrrolidon, Talkum, Stearinsäure und deren Salze wie auch langkettige Aliphaten, besonders bevorzugt getrocknete Stärke, mikrokristalline Cellulose oder Cellulosepulver und hydriertes Ricinus- oder Baumwollsaatöl eingesetzt.

Die Komplexe lassen sich auch durch Dispergierung in Fetten, Wachsen, festen, halbfesten oder flüssigen Kohlenwasserstoffen, Polyethylenglykolen, Emulgatoren etc. zu Salben, Cremes, Gelen, Pasten, Suppositorien und anderen halbfesten Arzneiformen verarbeiten. Es ist ebenfalls möglich, Sprays, Inhalate, Tampons oder Pflaster auf der Basis dieser Komplexe herzustellen.

Derartige Präparate sind bei Raumtemperatur über Jahre stabil.

Die erfindungsgemäßen Arzneimittel enthalten die an Crosspovidone, vorverkleisterte Stärke oder Dextran adsorbierten Prostaglandine in allgemeinen in Mengen von 0,001 bis 10 Gew.-%, vorzugsweise 0,01 bis 1 Gew.-%, besonders bevorzugt 0,03 bis 0,3 Gew.-%, gegebenenfalls neben den vorstehend aufgeführten Hilfsstoffen. Eine typische Arzneimittelformulierung weist folgende Zusammensetzung auf (Angaben in Gew.-%):

| | |
|---|---|
| Wirkstoff (Prostaglandin PGE): | 0,01 - 1 |
| Crosspovidone, vorverkleisterte Stärke, Dextran (alle 3 nachstehend als Adsorber bezeichnet) | 1 - 99,9 |
| Wasser: | 0,1 - 5 |

oder bei Verwendung von Hilfsstoffen:

a. Tablette:

| | | bevorzugt | besonders bevorzugt |
|---|---|---|---|
| Wirkstoff | 0,001 - 10 | 0,01 - 3 | 0,03 - 1 |
| Adsorber | 1 - 99 | 5 - 90 | 10 - 60 |
| Cellulose | 0 - 50 | 1 - 30 | 5 - 20 |
| Maisstärke | 0 - 50 | 1 - 30 | 5 - 20 |
| hydriertes Ricinusöl | 0 - 5 | 0,1 - 1 | 0,2 - 0,5 |
| Restwasser | 0,1 - 15 | 0,5 - 10 | 1 - 5 |

b. Kapsel:

| | | | bevorzugt | | besonders bevorzugt | | | | |
|---|---|---|---|---|---|---|---|---|---|
| Wirkstoff | 0,01 | - | 10 | 0,01 | - | 3 | 0,03 | - | 1 |
| Adsorber | 1 | - | 99 | 5 | - | 90 | 10 | - | 60 |
| Restwasser | 0,1 | - | 15 | 0,5 | - | 10 | 1 | - | 5 |
| Gelatine | 10 | - | 90 | 20 | - | 80 | 30 | - | 50 |

c. Pulver:

| | | | bevorzugt | | besonders bevorzugt | | | | |
|---|---|---|---|---|---|---|---|---|---|
| Wirkstoff | 0,001 | - | 10 | 0,01 | - | 3 | 0,03 | - | 1 |
| Adsorber | 1 | - | 99 | 5 | - | 90 | 10 | - | 60 |
| mikrokristalline Cellulose | 0 | - | 50 | 1 | - | 30 | 5 | - | 20 |
| vorverkleisterte Stärke | 0 | - | 50 | 1 | - | 30 | 5 | - | 20 |
| Restwasser | 0,1 | - | 15 | 0,5 | - | 10 | 1 | - | 5 |

Als vorzugsweise Formulierung werden Tabletten oder Kapseln im Sinne der vorliegenden Erfindung verwendet, die Prostaglandine vom E- Typ, bei den das C16-Atom substituiert ist, und Crosspovidone, vorverkleisterte Stärke oder Dextran enthalten. Bevorzugt hierbei werden Tabletten, die in den vorstehend genannten Zusammensetzungen verpreßt und bei Bedarf mit einer Hülle versehen werden. Vorzugsweise besteht die Kapselhülle aus Gelatine oder einem anderen, im Gastrointestinaltrakt löslichen Polymeren. Die Tabletten, wie auch die Kapseln, können zusätzlich mit einer weiteren Hülle oder auch einem Lack zur Verbesserung der Stabilität oder Handhabung oder auch zur Erzielung eines gewünschten Retardeffektes versehen werden. Damit die Anwendung des Mittels gezielt in Duodenal- und Dünndarmbereich erfolgen kann, kann ein magensaftresistenter Lack aufgebracht werden. Als Filmbildner können Cellulose-Derivate (bevorzugt: Methyl-, Ethyl-, Hydroxypropyl-, Hydroxypropylmethyl-, Celluloseacetatphthalat oder Hydroxypropylmethylcellulosephthalat), polymere Acrylate oder deren Copolymerisate mit anderen Substanzen, zumeist unter Zusatz von Weichmachern, verwendet werden. Die Hüllen können bei Bedarf mit gefärbten oder farbgebenden Stoffen versehen sein. Auch weitere Hilfsbzw. Geschmack- oder Wirkstoffe können sowohl in den Hüllen als auch in der Tablette bzw. Kapsel als auch in beiden vorhanden sein.

Auch bei den Tabletten- bzw. Kapselformulierungen sind die vorstehend angegebenen Bereiche über Wirkstoff/Adsorber-Verhältnis, Wassergehalt, Wirkstoffgehalt bevorzugte Bereiche, auch in jeder beliebigen Kombination der angegebenen Bereiche miteinander.

Erfindungsgemäß ist es auch möglich, mehrere Wirkstoffe von Prostaglandinen in dem gewünschten Verhältnis miteinander zu vermischen und der gewünschten Formulierung zuzuführen, wobei dann die Gew.-% sich auf den gesamten Wirkstoffgehalt beziehen und sich nicht additiv zusammenzusetzen. Analoges ist für die Adsorber gültig.

Erfindungsgemäß besonders bevorzugt auch für die Tabletten- bzw. Kapselformulierung wird das bereits vorstehend beschriebene 16-Methyl-1-11α, 16 RS-Trihydroxyprost-13E-en-9on der Formel (I).

Ferner gelten als bevorzugt im Sinne der vorliegenden Erfindung folgende Prostaglandine:

II 15-Deoxy-16-hydroxy-17-cyclobutano-$PGE_1$-methylester

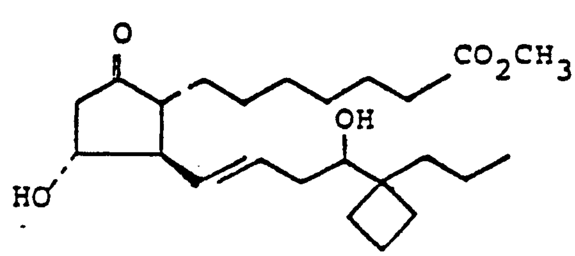

III $PGE_1$

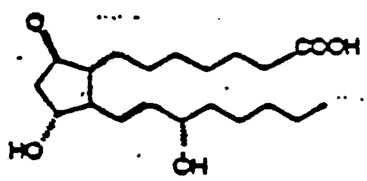

IV (17S)-17.20-Dimethyl-trans-$\Delta^2$ $PGE_1$

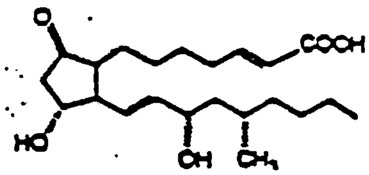

V 19β-Hydroxy-$PGE_1$

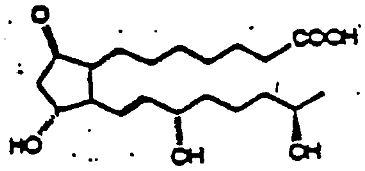

VI (±) (16RS)-15-Deoxy-16-hydroxy-16-methyl-$PGE_1$-methylester

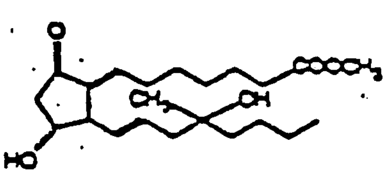

VII 16,16-Dimethyl-trans-$\Delta^2$-$PGE_1$-methylester

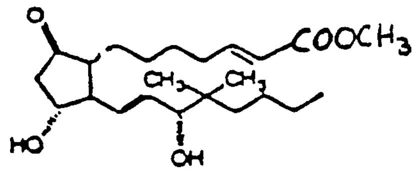

VIII 2-Decarboxy-2-hydroxy-methyl-$PGE_1$

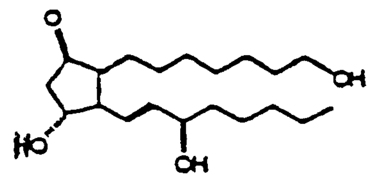

IX (+)-11α, 16α, β-Dihydroxy-1,9-dioxo-1-(hydroxy-methyl)-16-methyl-13-transprosiene

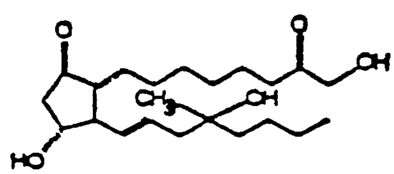

X 4,5,6-Trinor-3,7-inter-m-phenylen-3-oxa-$PGE_1$

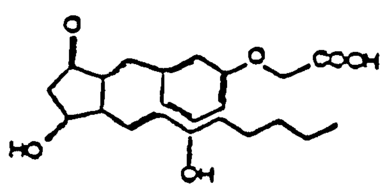

XI (15R)-15-Methyl-$PGE_2$

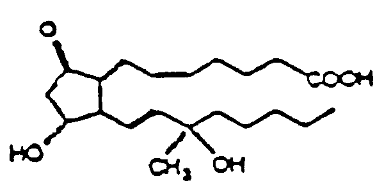

XII 16,16-Dimethyl-$PGE_2$

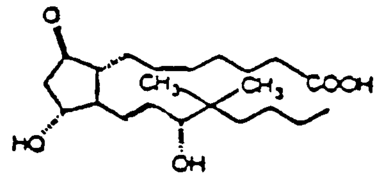

XIII 16-Methyl-20-methoxy-$PGE_2$

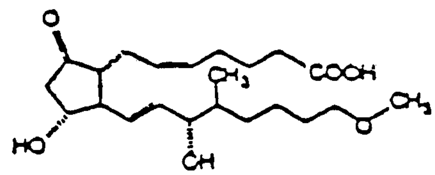

XIV (±) (16RS)-15-Deoxy-16-hydroxy-16-methyl-$PGE_2$-methylester

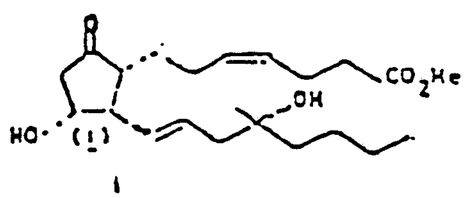

XV (+)-4,5-Didehydro-16-phenoxy-ω-tetranor-$PGE_1$-methylester

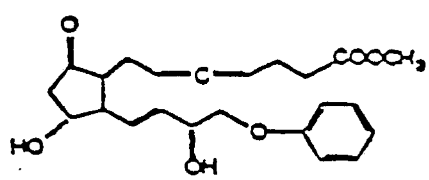

XVI N-Methan-sulfonyl-10-phenoxy-ω-tetranor-$PGE_2$-amid

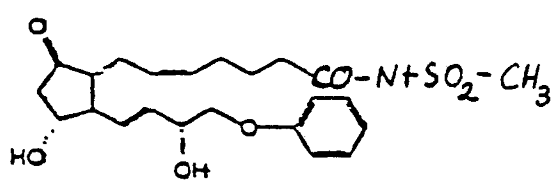

Die erfindungsgemäßen an Crosspovidone, vorverkleisterte Stärke oder Dextran gebundenen Prostaglandine, die nach dem erfindungsgemäßen Verfahren hergestellten Komplexe, die Formulierungen und Tabletten und Kapseln werden als Arzneimittel eingesetzt. Bevorzugt werden die erfindungsgemäßen Komplexe zur Bekämpfung von Magen-Darm-Ulcera, Hypertension, Asthma bronchiale, Thrombosen sowie bei der Einleitung von Wehen oder Aborten bei Menschen und Tieren, insbesondere bei Säugern, eingesetzt. Bevorzugt ist die Verwendung bei der Bekämpfung von Magen-Darm-Ulcera und bevorzugt in Form von Tabletten, Kapseln sowie Suppositorien. Eine besonders bevorzugte Anwendung stellt die Kombination Wirkstoff/Dextran in injizierbaren Formulierungen oder Infusions-Formulierungen dar.

Absorber bedeutet im folgenden Beispielteil jeweils Crosspovidone oder vorverkleisterte Stärke oder Dextran, wenn der Begriff Adsorber benutzt wird, bedeutet dies, daß die Beispiele mit den 3 Adsorbern Crosspovidone, vorverkleisterte Stärke und Dextran in den angegebenen Zusammensetzungen und Mengen durchgeführt werden.

**Beispiel 1**

5,0 g Wirkstoff der Formel I werden in 150 ml Ethanol und 450 ml Wasser gelöst.

995,0 g Adsorber werden in 5 l Wasser suspendiert und mit der obigen Lösung intensiv verrührt, in Schalen gegossen und mittels Gefriertrocknung getrocknet. Das Lyophilisat wird durch ein Sieb von 0,5 mm Maschenweite gesiebt und wie folgt weiterverarbeitet:

**A) Tabletten zu 0,1 mg Wirkstoff Formel I**

200 g Lyophilisat
300 g Cross-Povidone
200 g Cellulose Pulver
95 g getrocknete Maisstärke
5 g hydriertes Ricinusöl

werden gemischt und zu Tabletten von 80,0 mg Gewicht verpreßt.

**B) Kapseln zu 0,1 mg Wirkstoff Formel I**

200 g Lyophilisat
300 g Cross-Povidone

werden gemischt und diese Mischung zu 50,0 mg in Hartgelatine-Kapseln der Größe 4 abgefüllt.

**C) Pulver zu 0,3 mg Wirkstoff Formel I**

5000 g Maisstärke
3700 g mikrokristalline Cellulose
300 g vorverkleisterte Maisstärke

werden gemischt und mit Wasser granuliert. Das Granulat wird getrocknet und durch ein Sieb mit 0,8 mm Maschenweite gesiebt.

600 g Lyophilisat
400 g Cross-Povidone
9000 g des obigen Granulates

werden gemischt und zu 1,0 g in Beutel abgefüllt und diese verschlossen. Die Beutel können aus Papier, Aluminium-Folie oder einer indifferenten, für pharmazeutische Zwecke gegeigneten Polymer-Folie bestehen. Die Beutel können je nach Material, verklebt, verschweißt oder auf jede andere Methode, gegebenenfalls unter Luftauschluß und gegebenenfalls Inertbegasung verschlossen werden. Bei der Durchführung der Beispiele wurden Papierbeute, die verklebt wurden, verwendet.

**D) Creme 100 g enthaltend 15 mg Wirkstoff Formel I**

Ein Öl und eine Emulgatorphase bestehend aus:

| | |
|---|---|
| 2-Octyl-dodecanol | 120 g |
| Cetylstearylalkohol | 100 g |
| Walrat künstlich | 20 g |
| Sorbitanmonostearat | 15 g |
| Polyoxyethylen(20)-sorbitanmonostearat | 10 g |

werden auf 60°C erwärmt und geschmolzen. Dann werden 705 g Wasser zugegeben und bis zur Bildung einer homogenen Creme intensiv gewährt.

Nach dem Abkühlen werden 30 g Lyophilisat zugegeben und intensiv verrührt. Die fertige Creme wird zu 100 g in Tuben abgefüllt.

**E) Salbe 100 g enthaltend 15 mg Wirkstoff Formel I**

30 g Lyophilisat werden mit 150 g weißer Vaseline verrieben, bis eine homogene Masse entstanden ist. Diese wird dann mit 820 g weißer Vaseline intensiv zu einer Salbe verrührt und z. B. zu 100 g in Tuben abgefüllt.

**F) Suppositorien zu 0,5 mg Wirkstoff Formel I**

950 g Adeps solidus als Suppositorienmasse werden auf ca. 42°C erwärmt, 50 g Lyophilisat darin homogen suspendiert und in Suppositorienformen zu 2 g gegossen. Beim Abkühlen erstarrt die Masse zu den fertigen Suppositorien.

In den folgenden Tabellen 1 a, b und c werden Tablettenformulierungen für Wirkstoff der Formel I, 16-Methyl-1-11α, 16 RS-Trihydroxyprost-13E-en-9on als Komplexe an Crosspovidone, vorverkleisterter Stärke oder Dextran mit diversen Hilfsstoffen in den erfindungsgemäß bevorzugten Bereichen angegeben. Sämtliche Gewichtsangaben sind in mg.

**Tabelle 1a Beispiele für Tablettenformulierungen**

| Nr. | Formel I | Crosspovidone | Cellulose Pulver | microkrist. Cellulose | Maisstärke | Mannit | Polyvinylpyrrolidon | hydriertes Ricinusöl | Magnesiumstearat |
|---|---|---|---|---|---|---|---|---|---|
| 1 | 0,01 | 1,0 | 50 | - | 25 | - | 3,79 | 0,2 | - |
| 2 | 0,01 | 2,0 | - | 50 | 25 | - | 2,79 | 0,2 | - |
| 3 | 0,01 | 5,0 | 50 | - | 20 | - | 3,79 | - | 0,2 |
| 4 | 0,01 | 2,0 | 50 | - | 27,69 | 40 | - | 0,3 | - |
| 5 | 0,01 | 5,0 | - | 50 | 24,69 | 40 | - | - | 0,3 |
| 6 | 0,03 | 3,0 | 50 | - | 23,77 | - | 3,0 | 0,2 | - |
| 7 | 0,03 | 6,0 | - | 50 | 8,77 | 15 | - | - | 0,2 |
| 8 | 0,03 | 15,0 | 30 | - | 14,77 | 20 | - | 0,2 | - |
| 9 | 0,03 | 6,0 | - | 50 | 28,67 | 30 | 5,0 | 0,3 | - |
| 10 | 0,03 | 15,0 | 70 | - | 34,67 | - | - | - | 0,3 |
| 11 | 0,05 | 5,0 | 50 | - | 21,75 | - | 3,0 | 0,2 | - |
| 12 | 0,05 | 10,0 | - | 30 | 19,75 | 20 | - | - | 0,2 |
| 13 | 0,05 | 25,0 | 25 | - | 9,75 | 20 | - | 0,2 | - |
| 14 | 0,05 | 10,0 | 70 | - | 11,65 | 20 | - | 0,3 | - |
| 15 | 0,05 | 25,0 | - | 50 | 39,65 | - | 5,0 | - | 0,3 |
| 16 | 0,1 | 10,0 | 30 | - | 19,7 | 20 | - | 0,2 | - |
| 17 | 0,1 | 20,0 | 30 | - | 26,7 | - | 3,0 | - | 0,2 |
| 18 | 0,1 | 50,0 | - | 20 | 9,7 | - | 3,0 | - | 0,2 |
| 19 | 0,1 | 20,0 | 50 | - | 19,6 | 30 | - | 0,3 | - |
| 20 | 0,1 | 50,0 | - | 40 | 24,6 | - | 5,0 | - | 0,3 |
| 21 | 0,3 | 30,0 | 30 | - | 19,5 | - | - | 0,2 | - |
| 22 | 0,3 | 60,0 | - | 10 | 9,5 | - | - | - | 0,2 |
| 23 | 0,3 | 150,0 | - | 20 | 19,3 | 10 | - | - | 0,4 |
| 24 | 0,3 | 60,0 | 40 | - | 9,4 | 10 | - | 0,3 | - |
| 25 | 0,3 | 150,0 | 80 | - | 59,2 | - | 10 | - | 0,5 |
| 26 | 0,6 | 60,0 | 10 | - | 9,2 | - | - | 0,2 | - |
| 27 | 0,6 | 120,0 | 30 | - | 22,0 | 20 | 7,0 | - | 0,4 |
| 28 | 0,6 | 60,0 | 40 | - | 19,1 | - | - | 0,3 | - |
| 29 | 1,0 | 100,0 | - | 70 | 28,5 | - | - | - | 0,5 |

**Tabelle 1b Beispiele für Tablettenformulierungen**

| Nr. | Formel I | Maisstärke vorver-kleistert | Cellulose Pulver | microkrist. Cellulose | Maisstärke | Mannit | Cross-povidone | hydriertes Ricinusöl | Magnesium stearat |
|---|---|---|---|---|---|---|---|---|---|
| 1 | 0,01 | 1,0 | 50 | - | 28,79 | - | - | 0,2 | - |
| 2 | 0,01 | 2,0 | - | 50 | 27,79 | - | - | - | 0,2 |
| 3 | 0,01 | 5,0 | - | 50 | 19,79 | - | 5,0 | - | 0,2 |
| 4 | 0,01 | 2,0 | 60 | - | 27,69 | 30 | - | 0,3 | - |
| 5 | 0,01 | 5,0 | - | 60 | 24,69 | 30 | - | - | 0,3 |
| 6 | 0,03 | 3,0 | 50 | - | 26,77 | - | - | 0,2 | - |
| 7 | 0,03 | 6,0 | 50 | - | 18,77 | - | 5,0 | 0,2 | - |
| 8 | 0,03 | 15,0 | 30 | - | 4,77 | - | 30 | - | 0,2 |
| 9 | 0,03 | 6,0 | - | 60 | 23,67 | 30 | - | - | 0,3 |
| 10 | 0,03 | 15,0 | - | 60 | 14,67 | - | 30 | - | 0,3 |
| 11 | 0,05 | 5,0 | 50 | - | 19,75 | - | 5 | 0,2 | - |
| 12 | 0,05 | 10,0 | 50 | - | 9,75 | - | 10 | 0,2 | - |
| 13 | 0,05 | 25,0 | - | 20 | 24,65 | - | 50 | - | 0,3 |
| 14 | 0,05 | 10,0 | 50 | - | 29,65 | 20 | 10 | 0,3 | - |
| 15 | 0,05 | 5,0 | - | 50 | 24,65 | 30 | 10 | - | 0,3 |
| 16 | 0,1 | 10 | - | 50 | 9,7 | - | 10 | 0,2 | - |
| 17 | 0,1 | 20 | 40 | - | 19,6 | - | 40 | - | 0,3 |
| 18 | 0,1 | 50 | 60 | - | 39,5 | - | 150 | - | 0,4 |
| 19 | 0,1 | 10 | 60 | - | 39,6 | - | 10 | 0,3 | - |
| 20 | 0,1 | 20 | - | 100 | 39,5 | - | 40 | - | 0,4 |
| 21 | 0,3 | 30 | - | 20 | 9,4 | - | 60 | 0,3 | - |
| 22 | 0,3 | 60 | 10 | - | 9,3 | - | 120 | 0,4 | - |
| 23 | 0,3 | 150 | - | 100 | 48,7 | - | 300 | - | 1,0 |
| 24 | 0,3 | 30 | 100 | - | 9,3 | - | 60 | 0,4 | - |
| 25 | 0,3 | 60 | 60 | - | 29,2 | - | 150 | - | 0,5 |
| 26 | 0,6 | 60 | - | 10 | 9,0 | - | 120 | - | 0,4 |
| 27 | 0,6 | 120 | - | 120 | 58,4 | - | 300 | 1,0 | - |
| 28 | 0,6 | 60 | 60 | - | 28,9 | - | 150 | 0,5 | - |
| 29 | 1,0 | 100 | 150 | - | 48,8 | - | 300 | 1,0 | - |

**Tabelle 1c Beispiele für Tablettenformulierungen**

| Nr. | Formel I | Dextran | Cellulose-Pulver | mikrokrist. Cellulose | Maisstärke | Mannit | Cross-povidone | hydriertes Ricinusöl | Magnesium-stearat |
|---|---|---|---|---|---|---|---|---|---|
| 1 | 0,01 | 1,0 | 50 | - | 28,79 | - | - | 0,2 | - |
| 2 | 0,01 | 2,0 | - | 50 | 27,79 | - | - | - | 0,2 |
| 3 | 0,01 | 5,0 | - | 50 | 19,79 | - | 5,0 | - | 0,2 |
| 4 | 0,01 | 2,0 | 60 | - | 27,69 | 30 | - | 0,3 | - |
| 5 | 0,01 | 5,0 | - | 60 | 24,69 | 30 | - | - | 0,3 |
| 6 | 0,03 | 3,0 | 50 | - | 26,77 | - | - | 0,2 | - |
| 7 | 0,03 | 6,0 | 50 | - | 18,77 | - | 5,0 | 0,2 | - |
| 8 | 0,03 | 15,0 | 30 | - | 4,77 | - | 30,0 | - | 0,2 |
| 9 | 0,03 | 6,0 | - | 60 | 23,67 | 30 | - | - | 0,3 |
| 10 | 0,03 | 15,0 | - | 60 | 24,67 | - | 30,0 | - | 0,3 |
| 11 | 0,05 | 5,0 | 50 | - | 19,75 | - | 5,0 | 0,2 | - |
| 12 | 0,05 | 10,0 | 50 | - | 9,75 | - | 10,0 | 0,2 | - |
| 13 | 0,05 | 25,0 | - | 20 | 24,65 | - | 50,0 | - | 0,3 |
| 14 | 0,05 | 10,0 | 50 | - | 29,65 | 20 | 10,0 | 0,3 | - |
| 15 | 0,05 | 5,0 | - | 50 | 24,65 | 30 | 10,0 | - | 0,3 |
| 16 | 0,1 | 10,0 | - | 50 | 9,7 | - | 10,0 | 0,2 | - |
| 17 | 0,1 | 20,0 | 40 | - | 19,6 | - | 40,0 | - | 0,3 |
| 18 | 0,1 | 50 | 60 | - | 39,5 | - | 150,0 | - | 0,4 |
| 19 | 0,1 | 10,0 | 60 | - | 39,6 | - | 10,0 | 0,3 | - |
| 20 | 0,1 | 20,0 | - | 100 | 39,5 | - | 40,0 | - | 0,4 |
| 21 | 0,3 | 30,0 | - | 20 | 9,4 | - | 60,0 | 0,3 | - |
| 22 | 0,3 | 60,0 | 10 | - | 9,3 | - | 120 | 0,4 | - |
| 23 | 0,3 | 150,0 | - | 100 | 48,7 | - | 300 | - | 1,0 |
| 24 | 0,3 | 30,0 | 100 | - | 9,3 | - | 60 | 0,4 | - |
| 25 | 0,3 | 60,0 | 60 | - | 29,2 | - | 150 | - | 0,5 |
| 26 | 0,6 | 60,0 | - | 10 | 9,0 | - | 120 | - | 0,4 |
| 27 | 0,6 | 120,0 | - | 120 | 58,4 | - | 300 | 1,0 | - |
| 28 | 0,6 | 60,0 | 60 | - | 28,9 | - | 150 | 0,3 | - |
| 29 | 1,0 | 100,0 | 150 | - | 48,0 | - | 300 | 1,0 | - |

In den folgenden Tabellen 2 a, b und c werden Kapselformulierungen für Wirkstoff 16-Methyl-1,11α-16 RS-trihydroxyprost-13E-en-9on Formel I, als Komplex an die Adsorber Crosspovidone, vorverkleisterter Stärke, Dextran, gegebenenfalls unter Zusatz weiterer Hilfsstoffe, in mg angegeben.

**Tabelle 2a Beispiele für Kapselformlierungen von Wirkstoff der Formel I**

| Nr. | Formel I | Cross-Povidone | Cellulose-Pulver | Maisstärke | Lactose | Magnesium-stearat |
|---|---|---|---|---|---|---|
| 1 | 0,01 | 1,0 | 15 | 3,49 | 20 | 0,5 |
| 2 | 0,01 | 2,0 | 10 | 7,99 | 20 | - |
| 3 | 0,01 | 5,0 | 30 | 4,49 | 10 | 0,5 |
| 4 | 0,01 | 35,0 | - | 4,99 | - | - |
| 5 | 0,01 | 25,0 | 10 | 4,99 | - | - |
| 6 | 0,03 | 3,0 | 15 | 6,47 | 15 | 0,5 |
| 7 | 0,03 | 6,0 | 15 | 8,47 | 10 | 0,5 |
| 8 | 0,03 | 15,0 | 15 | 9,97 | - | - |
| 9 | 0,03 | 36,0 | - | 3,97 | - | - |
| 10 | 0,03 | 45,0 | 15 | 9,97 | - | - |
| 11 | 0,05 | 5,0 | 15 | 9,47 | 10 | 0,5 |
| 12 | 0,05 | 10,0 | 15 | 14,95 | - | - |
| 13 | 0,05 | 25,0 | 10 | 4,95 | - | - |
| 14 | 0,05 | 30,0 | 5 | 4,95 | - | - |
| 15 | 0,05 | 45,0 | 15 | 9,95 | - | - |
| 16 | 0,1 | 10,0 | 15 | 14,9 | - | - |
| 17 | 0,1 | 20,0 | 10 | 9,9 | - | - |
| 18 | 0,1 | 50,0 | 10 | 9,9 | - | - |
| 19 | 0,1 | 30,0 | 5 | 4,9 | - | - |
| 20 | 0,1 | 39,9 | - | - | - | - |
| 21 | 0,3 | 30,0 | 5 | 4,7 | - | - |
| 22 | 0,3 | 60,0 | 5 | 4,7 | - | - |
| 23 | 0,3 | 150,0 | - | - | - | - |
| 24 | 0,3 | 39,7 | - | - | - | - |
| 25 | 0,3 | 60,0 | 15 | 9,2 | 15 | 0,5 |

**Tabelle 2b Beispiele für Kapselformulierungen**

| Nr. | Formel I | Maisstärke vorver-kleistert | Cross-povidone | Cellulose pulver | Maisstärke |
|---|---|---|---|---|---|
| 1 | 0,01 | 1,0 | 20 | 10 | 8,99 |
| 2 | 0,01 | 2,0 | 20 | 10 | 7,99 |
| 3 | 0,01 | 5,0 | 20 | 10 | 4,99 |
| 4 | 0,01 | 2,0 | 20 | - | 17,99 |
| 5 | 0,01 | 5,0 | 20 | - | 14,99 |
| 6 | 0,03 | 3,0 | 20 | 10 | 6,97 |
| 7 | 0,03 | 6,0 | 20 | 10 | 3,97 |
| 8 | 0,03 | 15,0 | 20 | - | 4,97 |
| 9 | 0,03 | 6,0 | 20 | - | 13,97 |
| 10 | 0,03 | 15,0 | 30 | 20 | 4,97 |
| 11 | 0,05 | 5,0 | 20 | 10 | 4,95 |
| 12 | 0,05 | 10,0 | 20 | - | 9,95 |
| 13 | 0,05 | 25,0 | 45 | - | - |
| 14 | 0,05 | 10,0 | 30 | 20 | 9,95 |
| 15 | 0,05 | 25,0 | 50 | 20 | 4,95 |
| 16 | 0,1 | 10,0 | 20 | - | 9,9 |
| 17 | 0,1 | 20,0 | 40 | - | 9,9 |
| 18 | 0,1 | 50,0 | 100 | - | - |
| 19 | 0,1 | 10,0 | 30 | 20 | 9,9 |
| 20 | 0,1 | 20,0 | 60 | 10 | 9,9 |
| 21 | 0,3 | 30 | 60 | - | 9,7 |
| 22 | 0,3 | 60,0 | 120 | - | - |
| 23 | 0,3 | 150,0 | 300 | - | - |
| 24 | 0,3 | 30,0 | 80 | 20 | 19,7 |
| 25 | 0,3 | 60,0 | 150 | 10 | 19,7 |

**Tabelle 2c**

| Nr. | Formel I | Dextran | Cross-povidone | Cellulose pulver | Maisstärke |
|---|---|---|---|---|---|
| 1 | 0,01 | 1,0 | 20 | 10 | 8,99 |
| 2 | 0,01 | 2,0 | 20 | 10 | 7,99 |
| 3 | 0,01 | 5,0 | 20 | 10 | 4,99 |
| 4 | 0,01 | 2,0 | 20 | - | 17,99 |
| 5 | 0,01 | 5,0 | 20 | - | 14,99 |
| 6 | 0,03 | 3,0 | 20 | 10 | 6,97 |
| 7 | 0,03 | 6,0 | 20 | 10 | 3,97 |
| 8 | 0,03 | 15,0 | 20 | - | 4,97 |
| 9 | 0,03 | 6,0 | 20 | - | 13,97 |
| 10 | 0,03 | 15,0 | 30 | 20 | 4,97 |
| 11 | 0,05 | 5,0 | 20 | 10 | 4,95 |
| 12 | 0,05 | 10,0 | 20 | - | 9,95 |
| 13 | 0,05 | 25,0 | 45 | - | - |
| 14 | 0,05 | 10,0 | 30 | 20 | 9,95 |
| 15 | 0,05 | 25,0 | 50 | 20 | 4,95 |
| 16 | 0,1 | 10,0 | 20 | - | 9,9 |
| 17 | 0,1 | 20,0 | 40 | - | 9,9 |
| 18 | 0,1 | 50,0 | 100 | - | - |
| 19 | 0,1 | 10,0 | 30 | 20 | 9,9 |
| 20 | 0,1 | 20,0 | 60 | 10 | 9,9 |
| 21 | 0,3 | 30,0 | 60 | - | 9,7 |
| 22 | 0,3 | 60,0 | 120 | - | - |
| 23 | 0,3 | 150,0 | 300 | - | - |
| 24 | 0,3 | 30,0 | 80 | 20 | 19,7 |
| 25 | 0,3 | 60,0 | 150 | 20 | 19,7 |

In den folgenden Tabellen 3 bis 19 werden Tablettenformulierungen für verschiedene Wirkstoffe angegeben. Sämtliche Angaben in mg.
Als Wirkstoffe in den einzelnen Tabletten wurden folgende eingesetzt:

| Tabelle | Name | Formel |
|---|---|---|
| 3 | 15-Deoxy-16-hydroxy-17-cyclobutano-$PGE_1$-methylester | II |
| 4 | $PGE_1$ | III |
| 5 | (17S)-17,20-Dimethyl-trans-$\Delta^2$ $PGE_1$ | IV |
| 6 | 19β-Hydroxy-$PGE_1$ | V |
| 7 | (±) (16RS)-15-Deoxy-16-hydroxy-16-methyl-$PGE_1$-methylester | VI |
| 8 | 16,16-Dimethyl-trans-$\Delta^2$-$PGE_1$-methylester | VII |
| 9 | 2-Decarboxy-2-hydroxy-methyl-$PGE_1$ | VIII |
| 10 | (+)-11α, 16α, β-Dihydroxy-1,9-dioxo-1-(hydroxymethyl)-16-methyl-13-transprosiene | IX |
| 11 | 4,5,6-Trinor-3,7-inter-m-phenylen-3-oxa-$PGE_1$ | X |
| 12 | (15R)-15-methyl-$PGE_2$ | XI |
| 13 | 16,16-Dimethyl-$PGE_2$ | XII |
| 14 | 16-Methyl-20-methoxy-$PGE_2$ | XIII |
| 15 | (±) (16RS)15-Deoxy-16-hydroxy-16-methyl-$PGE_2$-methylester | XIV |
| 16 | (+)-4,5-Didehydro-16-phenoxy-ω-tetranor-$PGE_1$-methylester | XV |
| 17 | N-methan-sulfonyl-10-phenoxy-ω-tetranor-$PGE_2$-amid | XVI |

**Tabelle 3a Beispiele für Tablettenformulierungen**

| Nr. | Formel II | Crosspovidone | Cellulose Pulver | microkrist. Cellulose | Maisstärke | Mannit | Polyvinylpyrrolidon | hydriertes Ricinusöl | Magnesiumstearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 3b Beispiele for Tablettenformulierungen**

| Nr. | Formel II | Maisstärke vorverkleistert | Cellulose-Pulver | mikrokrist. Cellulose | Maisstärke | Mannit | Crosspovidone | hydriertes Ricinusöl | Magnesiumstearat |
|---|---|---|---|---|---|---|---|---|---|
| 1 | 0,01 | 2,0 | - | 50 | 27,79 | - | - | - | 0,2 |
| 2 | 0,03 | 6,0 | 50 | - | 18,77 | - | 5,0 | 0,2 | - |
| 3 | 0,1 | 20,0 | 40 | - | 19,6 | - | 40,0 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 3c Beispiele für Tablettenformulierungen**

| Nr. | Formel II | Dextran | Cellulose-Pulver | mikrokrist. Cellulose | Maisstärke | Polyvinylpyrrolidon | hydriertes Ricinusöl | Magnesiumstearat |
|---|---|---|---|---|---|---|---|---|
| 1 | 0,01 | 2,0 | 50 | - | 27,79 | - | - | 0,2 |
| 2 | 0,03 | 6,0 | 50 | - | 18,77 | 5,0 | - | 0,2 |
| 3 | 0,1 | 20,0 | - | 40 | 19,6 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 4a Beispiele für Tablettenformulierungen**

| Nr. | Formel III | Crosspovidone | Cellulose Pulver | mikrokrist. Cellulose | Maisstärke | Mannit | Polyvinylpyrrolidon | hydriertes Ricinusöl | Magnesiumstearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 4b Beispiele für Tablettenformulierungen**

| Nr. | Formel III | Maisstärke vorverkleistert | Cellulose-Pulver | mikrokrist. Cellulose | Maisstärke | Mannit | Crosspovidone | hydriertes Ricinusöl | Magnesiumstearat |
|---|---|---|---|---|---|---|---|---|---|
| 1 | 0,01 | 2,0 | - | 50 | 27,79 | - | - | - | 0,2 |
| 2 | 0,03 | 8,0 | 50 | - | 18,77 | - | 5,0 | 0,2 | - |
| 3 | 0,1 | 20,0 | 40 | - | 19,6 | - | 40,0 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 4c Beispiele für Tablettenformulierungen**

| Nr | Formel III | Dextran | Cellulose-Pulver | mikrokrist. Cellulose | Maisstärke | Polyvinylpyrrolidon | hydriertes Ricinusöl | Magnesiumstearat |
|---|---|---|---|---|---|---|---|---|
| 1 | 0,01 | 2,0 | 50 | - | 27,79 | - | - | 0,2 |
| 2 | 0,03 | 6,0 | 50 | - | 18,77 | 5,0 | - | 0,2 |
| 3 | 0,1 | 20,0 | - | 40 | 19,8 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 5a Beispiele für Tablettenformulierungen**

| Nr. | Formel IV | Crosspovidone | Cellulose Pulver | mikrokrist. Cellulose | Maisstärke | Mannit | Polyvinylpyrrolidon | hydriertes Ricinusöl | Magnesiumstearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 5b Beispiele für Tablettenformulierungen**

| Nr. | Formel IV | Maisstärke vorverkleistert | Cellulose-Pulver | mikrokrist. Cellulose | Maisstärke | Mannit | Crosspovidone | hydriertes Ricinusöl | Magnesiumstearat |
|---|---|---|---|---|---|---|---|---|---|
| 1 | 0,01 | 2,0 | - | 50 | 27,79 | - | - | - | 0,2 |
| 2 | 0,03 | 6,0 | 50 | - | 18,77 | - | 5,0 | 0,2 | - |
| 3 | 0,1 | 20,0 | 40 | - | 19,6 | - | 40,0 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 5c Beispiele für Tablettenformulierungen**

| Nr. | Formel IV | Dextran | Cellulose-Pulver | mikrokrist. Cellulose | Maisstärke | Polyvinylpyrrolidon | hydriertes-Ricinusöl | Magnesium stearat |
|---|---|---|---|---|---|---|---|---|
| 1 | 0,01 | 2,0 | 50 | - | 27,79 | - | - | 0,2 |
| 2 | 0,03 | 6,0 | 50 | - | 18,77 | 5,0 | - | 0,2 |
| 3 | 0,1 | 20,0 | - | 40 | 19,6 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 6a Beispiele für Tablettenformulierungen**

| Nr. | Formel V | Crosspovidone | Cellulose Pulver | mikrokrist. Cellulose | Maisstärke | Mannit | Polyvinylpyrrolidon | hydriertes Ricinusöl | Magnesiumstearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 6b Beispiele für Tablettenformulierungen**

| Nr. | Formel V | Maisstärke vorver-kleistert | Cellulose-Pulver | mikrokrist. Cellulose | Maisstärke | Mannit | Cross-povidone | hydriertes Ricinusöl | Magnesium-stearat |
|---|---|---|---|---|---|---|---|---|---|
| 1 | 0,01 | 2,0 | - | 50 | 27,79 | - | - | - | 0,2 |
| 2 | 0,03 | 6,0 | 50 | - | 18,77 | - | 5,0 | 0,2 | - |
| 3 | 0,1 | 20,0 | 40 | - | 19,6 | - | 40,0 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 6c Beispiele für Tablettenformulierungen**

| Nr. | Formel V | Dextran | Cellulose-Pulver | mikrokrist. Cellulose | Maisstärke | Polyvinyl-pyrrolidon | hydriertes Ricinusöl | Magnesium-stearat |
|---|---|---|---|---|---|---|---|---|
| 1 | 0,01 | 2,0 | 50 | - | 27,79 | - | - | 0,2 |
| 2 | 0,03 | 6,0 | 50 | - | 18,77 | 5,0 | - | 0,2 |
| 3 | 0,1 | 20,0 | - | 40 | 19,6 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 7a Beispiele für Tablettenformulierungen**

| Nr. | Formel VI | Cross-povidone | Cellulose Pulver | mikrokrist. Cellulose | Maisstärke | Mannit | Polyvinyl-pyrrolidon | hydriertes Ricinusöl | Magnesium-stearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 7b Beispiele für Tablettenformulierungen**

| Nr. | Formel VI | Maisstärke vorver-kleistert | Cellulose-Pulver | mikrokristall. Cellulose | Maisstärke | Mannit | Cross-povidone | hydriertes Ricinusöl | Magnesium-stearat |
|---|---|---|---|---|---|---|---|---|---|
| 1 | 0,01 | 2,0 | - | 50 | 27,79 | - | - | - | 0,2 |
| 2 | 0,03 | 6,0 | 50 | - | 18,77 | - | 5,0 | 0,2 | - |
| 3 | 0,1 | 20,0 | 40 | - | 19,8 | - | 40,0 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 7c Beispiele für Tablettenformulierungen**

| Nr. | Formel VI | Dextran | Cellulose-Pulver | mikrokrist. Cellulose | Maisstärke | Polvinyl-pyrrolidon | hydriertes Ricinusöl | Magnesium-stearat |
|---|---|---|---|---|---|---|---|---|
| 1 | 0,01 | 2,0 | 50 | - | 27,79 | - | - | 0,2 |
| 2 | 0,03 | 8,0 | 50 | - | 18,77 | 5,0 | - | 0,2 |
| 3 | 0,1 | 20,0 | - | 40 | 19,6 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 8a Beispiele für Tablettenformulierungen**

| Nr. | Formel VII | Crosspovidone | Cellulose Pulver | mikrokrist. Cellulose | Maisstärke | Mannit | Polyvinylpyrrolidon | hydriertes Ricinusöl | Magnesiumstearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 8b Beispiele für Tablettenformulierungen**

| Nr. | Formel VII | Maisstärke vorverkleistert | Cellulose-Pulver | mikrokrist. Cellulose | Maisstärke | Mannit | Crosspovidone | hydriertes Ricinusöl | Magnesiumstearat |
|---|---|---|---|---|---|---|---|---|---|
| 1 | 0,01 | 2,0 | - | 50 | 27,79 | - | - | - | 0,2 |
| 2 | 0,03 | 6,0 | 50 | - | 18,77 | - | 5,0 | 0,2 | - |
| 3 | 0,1 | 20,0 | 40 | - | 19,6 | - | 40,0 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 8c Beispiele für Tablettenformulierungen**

| Nr. | Formel VII | Dextran | Cellulose-Pulver | mikrokrist. Cellulose | Maisstärke | Polyvinylpyrrolidon | hydriertes Ricinusöl | Magnesiumstearat |
|---|---|---|---|---|---|---|---|---|
| 1 | 0,01 | 2,0 | 50 | - | 27,79 | - | - | 0,2 |
| 2 | 0,03 | 6,0 | 50 | - | 18,77 | 5,0 | - | 0,2 |
| 3 | 0,1 | 20,0 | - | 40 | 19,6 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 9a Beispiele für Tablettenformulierunmgen**

| Nr. | Formel VIII | Crosspovidone | Cellulose Pulver | mikrokrist. Cellulose | Maisstärke | Mannit | Polyvinylpyrrolidon | hydriertes Ricinusöl | Magnesiumstearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 9b Beispiele für Tablettenformulierungen**

| Nr. | Formel VIII | Maisstärke vorverkleistert | Cellulose-Pulver | mikrokrist. Cellulose | Maisstärke | Mannit | Crosspovidone | hydriertes Ricinusöl | Magnesiumstearat |
|---|---|---|---|---|---|---|---|---|---|
| 1 | 0,01 | 2,0 | - | 50 | 27,79 | - | - | - | 0,2 |
| 2 | 0,03 | 6,0 | 50 | - | 18,77 | - | 5,0 | 0,2 | - |
| 3 | 0,1 | 20,0 | 40 | - | 19,6 | - | 40,0 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 9c Beispiele für Tablettenformulierungen**

| Nr. | Formel VIII | Dextran | Cellulose-Pulver | mikrokrist. Cellulose | Maisstärke | Polyvinyl-pyrrolidon | hydriertes Ricinusöl | Magnesium-stearat |
|---|---|---|---|---|---|---|---|---|
| 1 | 0,01 | 2,0 | 50 | - | 27,79 | - | - | 0,2 |
| 2 | 0,03 | 6,0 | 50 | - | 18,77 | 5,0 | - | 0,2 |
| 3 | 0,1 | 20,0 | - | 40 | 19,6 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 10a Beispiele für Tablettenformulierungen**

| Nr. | Formel IX | Cross-povidone | Cellulose Pulver | mikrokrist. Cellulose | Maisstärke | Mannit | Polyvinyl-pyrrolidon | hydriertes Ricinusöl | Magnesium-stearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 10b Beispiele für Tablettenformulierungen**

| Nr. | Formel IX | Maisstärke vorver-kleistert | Cellulose-Pulver | mikrokrist. Cellulose | Maisstäke | Mannit | Cross-povidone | hydriertes Ricinusöl | Magnesium-stearat |
|---|---|---|---|---|---|---|---|---|---|
| 1 | 0,01 | 2,0 | - | 50 | 27,79 | - | - | - | 0,2 |
| 2 | 0,03 | 6,0 | 50 | - | 18,77 | - | 5,0 | 0,2 | - |
| 3 | 0,1 | 20,0 | 40 | - | 19,6 | - | 40,0 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 10c Beispiele für Tablettenformulierungen**

| Nr. | Formel IX | Dextran | Cellulose-Pulver | mikrokrist. Cellulose | Maisstärke | Polyvinyl-pyrrolidon | hydriertes Ricinusöl | Magneesium-stearat |
|---|---|---|---|---|---|---|---|---|
| 1 | 0,01 | 2,0 | 50 | - | 27,79 | - | - | 0,2 |
| 2 | 0,03 | 6,0 | 50 | - | 18,77 | 5,0 | - | 0,2 |
| 3 | 0,1 | 20,0 | - | 40 | 19,6 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 11a Beispiele für Tablettenformulierungen**

| Nr. | Formel X | Cross-povidone | Cellulose Pulver | mikrokrist. Cellulose | Maisstärke | Mannit | Polyvinyl-pyrrolidon | hydriertes Ricinusöl | Magnesium-stearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 11b Beispiele für Tablettenformulierungen**

| Nr. | Formel X | Maisstärke vorver-kleistert | Cellulose-Pulver | mikrokrist. Cellulose | Maisstärke | Mannit | Cross-povidone | hydriertes Ricinusöl | Magnesium-stearat |
|---|---|---|---|---|---|---|---|---|---|
| 1 | 0,01 | 2,0 | - | 50 | 27,79 | - | - | - | 0,2 |
| 2 | 0,03 | 6,0 | 50 | - | 18,77 | - | 5,0 | 0,2 | - |
| 3 | 0,1 | 20,0 | 40 | - | 19,6 | - | 40,8 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 11c Beispiele für Tablettenformulierungen**

| Nr. | Formel X | Dextran | Cellulose-Pulver | mikrokrist. Cellulose | Maisstärke | Polyvinyl-pyrrolidon | hydriertes Ricinusöl | Magnesium-stearat |
|---|---|---|---|---|---|---|---|---|
| 1 | 0,01 | 2,0 | 50 | - | 27,79 | - | - | 0,2 |
| 2 | 0,03 | 6,0 | 50 | - | 18,77 | 5,0 | - | 0,2 |
| 3 | 0,1 | 20,0 | - | 40 | 19,6 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 12a Beispiele für Tablettenformulierungen**

| Nr. | Formel XI | Cross-povidone | Cellulose Pulver | mikrokrist. Cellulose | Maisstärke | Mannit | Polyvinyl-pyrrolidon | hydriertes Ricinusöl | Magnesium-stearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 12b Beispiele für Tablettenformulierungen**

| Nr. | Formel XI | Maisstärke vorver-kleistert | Cellulose-Pulver | mikrokrist. Cellulose | Maisstärke | Mannit | Cross-povidone | hydriertes Ricinusöl | Magnesium-stearat |
|---|---|---|---|---|---|---|---|---|---|
| 1 | 0,01 | 2,0 | - | 50 | 27,79 | - | - | - | 0,2 |
| 2 | 0,03 | 6,0 | 50 | - | 18,77 | - | 5,0 | 0,2 | - |
| 3 | 0,1 | 20,0 | 40 | - | 19,6 | - | 40,0 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 12c Beispiele für Tablettenformulierungen**

| Nr. | Formel XI | Dextran | Cellulose-Pulver | mikrokrist. Cellulose | Maisstärke | Polyvinyl-pyrrolidon | hydriertes Ricinusöl | Magnesium-stearat |
|---|---|---|---|---|---|---|---|---|
| 1 | 0,01 | 2,0 | 50 | - | 27,79 | - | - | 0,2 |
| 2 | 0,03 | 6,0 | 50 | - | 18,77 | 5,0 | - | 0,2 |
| 3 | 0,1 | 20,0 | - | 40 | 19,6 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 13a Beispiele für Tablettenformulierungen**

| Nr. | Formel XII | Cross-povidone | Cellulose Pulver | mikrokrist. Cellulose | Maisstärke | Mannit | Polyvinyl-pyrrolidon | hydriertes Ricinusöl | Magnesium-stearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 13b Beispiele für Tablettenformulierungen**

| Nr. | Formel XII | Maisstärke vorver-kleistert | Cellulose-Pulver | mikrokrist. Cellulose | Maisstärke | Mannit | Cross-povidone | hydriertes Ricinusöl | Magnesium-stearat |
|---|---|---|---|---|---|---|---|---|---|
| 1 | 0,01 | 2,0 | - | 50 | 27,79 | - | - | - | 0,2 |
| 2 | 0,03 | 6,0 | 50 | - | 18,77 | - | 5,0 | 0,2 | - |
| 3 | 0,1 | 20,0 | 40 | - | 19,6 | - | 40,0 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 13c Beispiele für Tablettenformulierungen**

| Nr. | Formel XII | Dextran | Cellulose-Pulver | mikrokrist. Cellulose | Maisstärke | Polyvinyl-pyrrolidon | hydriertes Ricinusöl | Magnesium-stearat |
|---|---|---|---|---|---|---|---|---|
| 1 | 0,01 | 2,0 | 50 | - | 27,79 | - | - | 0,2 |
| 2 | 0,03 | 6,0 | 50 | - | 18,77 | 5,0 | - | 0,2 |
| 3 | 0,1 | 20,0 | - | 40 | 19,6 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 14a Beispiele für Tablettenformulierungen**

| Nr. | Formel XIII | Cross-povidone | Cellulose Pulver | mikrokrist. Cellulose | Maisstärke | Mannit | Polyvinyl-pyrrolidon | hydriertes Ricinusöl | Magnesium-stearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 14b Beispiele für Tablettenformulierungen**

| Nr. | Formel XIII | Maisstärke vorver-kleistert | Cellulose-Pulver | mikrokrist. Cellulose | Maisstärke | Mannit | Cross-povidone | hydriertes Ricinusöl | Magnesium-stearat |
|---|---|---|---|---|---|---|---|---|---|
| 1 | 0,01 | 2,0 | - | 50 | 27,79 | - | - | - | 0,2 |
| 2 | 0,03 | 6,0 | 50 | - | 18,77 | - | 5,0 | 0,2 | - |
| 3 | 0,1 | 20,0 | 40 | - | 19,6 | - | 40,0 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 14c Beispiele für Tablettenformulierungen**

| Nr. | Formel XIII | Dextran | Cellulose-Pulver | mikrokrist. Cellulose | Maisstärke | Polyvinylpyrrolidon | hydriertes Ricinusöl | Magnesiumstearat |
|---|---|---|---|---|---|---|---|---|
| 1 | 0,01 | 2,0 | 50 | - | 27,79 | - | - | 0,2 |
| 2 | 0,03 | 6,0 | 50 | - | 18,77 | 5,0 | - | 0,2 |
| 3 | 0,1 | 20,0 | - | 40 | 19,6 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 15a Beispiele für Tablettenformulierungen**

| Nr. | Formel XIV | Crosspovidone | Cellulose Pulver | mikrokrist. Cellulose | Maisstärke | Mannit | Polyvinylpyrrolidon | hydriertes Ricinusöl | Magnesiumstearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 15b Beispiele für Tablettenformulierungen**

| Nr. | Formel XIV | Maisstärke vorverkleistert | Cellulose-Pulver | mikrokrist. Cellulose | Maisstärke | Mannit | Crosspovidone | hydriertes Ricinusöl | Magnesiumstearat |
|---|---|---|---|---|---|---|---|---|---|
| 1 | 0,01 | 2,0 | - | 50 | 27,79 | - | - | - | 0,2 |
| 2 | 0,03 | 6,0 | 50 | - | 18,77 | - | 5,0 | 0,2 | - |
| 3 | 0,1 | 20,0 | 40 | - | 19,6 | - | 40,0 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 15c Beispiele für Tablettenformulierungen**

| Nr. | Formel XIV | Dextran | Cellulose-Pulver | mikrokrist. Cellulose | Maisstärke | Polyvinylpyrrolidon | hydriertes Ricinusöl | Magnesiumstearat |
|---|---|---|---|---|---|---|---|---|
| 1 | 0,01 | 2,0 | 50 | - | 27,79 | - | - | 0,2 |
| 2 | 0,03 | 6,0 | 50 | - | 18,77 | 5,0 | - | 0,2 |
| 3 | 0,1 | 20,0 | - | 40 | 19,6 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 16a Beispiele für Tablettenformulierungen**

| Nr. | Formel XV | Crosspovidone | Cellulose Pulver | mikrokrist. Cellulose | Maisstärke | Mannit | Polyvinylpyrrolidon | hydriertes Ricinusöl | Magnesiumstearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 16b Beispiele für Tablettenformulierungen**

| Nr. | Formel XV | Dextran | Cellulose-Pulver | mikrokrist. Cellulose | Maisstärke | Polyvinylpyrrolidon | hydriertes Ricinusöl | Magnesiumstearat |
|---|---|---|---|---|---|---|---|---|
| 1 | 0,01 | 2,0 | 50 | - | 27,79 | - | - | 0,2 |
| 2 | 0,03 | 6,0 | 50 | - | 18,77 | 5,0 | - | 0,2 |
| 3 | 0,1 | 20,0 | - | 40 | 19,6 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 16c Beispiele für Tabletteformulierungen**

| Nr. | Formel XV | Maisstärke vorverkleistert | Cellulose-Pulver | mikrokrist. Cellulose | Makisstärke | Mannit | Crosspovidone | hydriertes Ricinusöl | Magnesiumstearat |
|---|---|---|---|---|---|---|---|---|---|
| 1 | 0,01 | 2,0 | - | 50 | 27,79 | - | - | - | 0,2 |
| 2 | 0,03 | 6,0 | 50 | - | 18,77 | - | 5,0 | 0,2 | - |
| 3 | 0,1 | 20,0 | 40 | - | 19,6 | - | 40,0 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 17a Beispiele für Tablettenformulierungen**

| Nr. | Formel XVI | Crosspovidone | Cellulose Pulver | mikrokrist. Cellulose | Maisstärke | Mannit | Polyvinylpyrrolidon | hydriertes Ricinusöl | Magnesiumstearat |
|---|---|---|---|---|---|---|---|---|---|
| 1 | 0,01 | 2,0 | 50 | - | 24,79 | - | 3,0 | 0,2 | - |
| 2 | 0,03 | 6,0 | - | 50 | 8,77 | 15 | - | - | 0,2 |
| 3 | 0,1 | 20,0 | 30 | - | 26,7 | - | 3,0 | 0,2 | - |
| 4 | 0,3 | 60,0 | 40 | - | 1,4 | 10 | - | 0,3 | - |
| 5 | 1,0 | 200,0 | - | 70 | 28,5 | - | - | - | 0,5 |

**Tabelle 17b Beispiele für Tablettenformulierungen**

| Nr. | Formel XVI | Maisstärke vorverkleistert | Cellulose-Pulver | mikrokrist. Cellulose | Maisstärke | Mannit | Crosspovidone | hydriertes Ricinusöl | Magnesiumstearat |
|---|---|---|---|---|---|---|---|---|---|
| 1 | 0,01 | 2,0 | - | 50 | 27,79 | - | - | - | 0,2 |
| 2 | 0,03 | 6,0 | 50 | - | 18,77 | - | 5,0 | 0,2 | - |
| 3 | 0,1 | 20,0 | 40 | - | 19,6 | - | 40,0 | - | 0,3 |
| 4 | 0,3 | 60,0 | 10 | - | 9,3 | - | 120,0 | 0,4 | - |
| 5 | 1,0 | 180,0 | 150 | - | 48,0 | - | 300,0 | 1,0 | - |

**Tabelle 17c Beispiele für Tablettenformulierungen**

| Nr. | Formel XVI | Dextran | Cellulose-Pulver | mikrokrist. Cellulose | Maisstärke | Polyvinylpyrrolidon | hydriertes Ricinusöl | Magnesiumstearat |
|---|---|---|---|---|---|---|---|---|
| 1 | 0,01 | 2,0 | 50 | - | 27,79 | - | - | 0,2 |
| 2 | 0,03 | 6,0 | 50 | - | 18,77 | 5,0 | - | 0,2 |
| 3 | 0,1 | 20,0 | - | 40 | 19,6 | 40,0 | 0,3 | - |
| 4 | 0,3 | 60,0 | - | 10 | 9,3 | 120,0 | 0,4 | - |
| 5 | 1,0 | 100,0 | 150 | - | 48,0 | 300,0 | - | 1,0 |

**Tabelle 18a Beispiel für Kapselformulierungen**

| Nr. | Formel II | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 18b Beispiel für Kapselformulierungen**

| Nr. | Formel II | Maisstärke vorver-kleistert | Cross-povidone | Cellulose-Pulver | Maisstärke |
|---|---|---|---|---|---|
| 1 | 0,01 | 2,0 | 20 | 10 | 7,99 |
| 2 | 0,03 | 6,0 | 20 | 10 | 3,79 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 18c Beispiel für Kapselformulierungen**

| Nr. | Formel II | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,97 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 19a Beispiel für Kapselformulierungen**

| Nr. | Formel III | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 19b Beispiel für Kapselformulierungen**

| Nr. | Formel III | Maisstärke vorver-kleistert | Cross-povidone | Cellulose-Pulver | Maisstärke |
|---|---|---|---|---|---|
| 1 | 0,01 | 2,0 | 20 | 10 | 7,99 |
| 2 | 0,03 | 6,0 | 20 | 10 | 3,79 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 19c Beispiel für Kapselformulierungen**

| Nr. | Formel III | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,97 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 20a Beispiel für Kapselformulierungen**

| Nr. | Formel IV | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 20b Beispiel für Kapselformulierungen**

| Nr. | Formel IV | Maisstärke vorver-kleistert | Cross-povidone | Cellulose-Pulver | Maisstärke |
|---|---|---|---|---|---|
| 1 | 0,01 | 2,0 | 20 | 10 | 7,9 |
| 2 | 0,03 | 6,0 | 20 | 10 | 3,79 |
| 3 | 0,05 | 10,0 | 20 | - | 9,9 |
| 4 | 0,1 | 20,0 | 40 | - | 9,9 |
| 5 | 0,3 | 60,0 | 120 | - | - |

**Tabelle 20c Beispiel für Kapselformulierungen**

| Nr. | Formel IV | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,97 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 21a Beispiel für Kapselformulierungen**

| Nr. | Formel V | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 21b Beispiel für Kapselformulierungen**

| Nr. | Formel V | Maisstärke vorver-kleistert | Cross-povidone | Cellulose-Pulver | Maisstärke |
|---|---|---|---|---|---|
| 1 | 0,01 | 2,0 | 20 | 10 | 7,99 |
| 2 | 0,03 | 6,0 | 20 | 10 | 3,79 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 21c Beispiel für Kapselformulierungen**

| Nr. | Formel V | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,97 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 22a Beispiel für Kapselformulierungen**

| Nr. | Formel VI | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 22b Beispiel für Kapselformulierungen**

| Nr. | Formel VI | Maisstärke vorver-kleistert | Cross-povidone | Cellulose-Pulver | Maisstärke |
|---|---|---|---|---|---|
| 1 | 0,01 | 2,0 | 20 | 10 | 7,99 |
| 2 | 0,03 | 6,0 | 20 | 10 | 3,79 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 22c Beispiel für Kapselformulierungen**

| Nr. | Formel VI | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,97 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 23a Beispiel für Kapselformulierungen**

| Nr. | Formel VII | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 23b Beispiel für Kapselformulierungen**

| Nr. | Formel VII | Maisstärke vorver-kleistert | Cross-povidone | Cellulose-Pulver | Maisstärke |
|---|---|---|---|---|---|
| 1 | 0,01 | 2,0 | 20 | 10 | 7,99 |
| 2 | 0,03 | 6,0 | 20 | 10 | 3,79 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 23c Beispiel für Kapselformulierungen**

| Nr. | Formel VII | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,97 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 24a Beispiel für Kapselformulierungen**

| Nr. | Formel VIII | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 24b Beispiel für Kapselformulierungen**

| Nr. | Formel VIII | Maisstärke vorver-kleistert | Cross-povidone | Cellulose-Pulver | Maisstärke |
|---|---|---|---|---|---|
| 1 | 0,01 | 2,0 | 20 | 10 | 7,99 |
| 2 | 0,03 | 6,0 | 20 | 10 | 3,79 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 24c Beispiel für Kapselformulierungen**

| Nr. | Formel VIII | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,97 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 25a Beispiel für Kapselformulierungen**

| Nr. | Formel IX | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 25b Beispiel für Kapselformulierungen**

| Nr. | Formel IX | Maisstärke vorver-kleistert | Cross-povidone | Cellulose-Pulver | Maisstärke |
|---|---|---|---|---|---|
| 1 | 0,01 | 2,0 | 20 | 10 | 7,99 |
| 2 | 0,03 | 6,0 | 20 | 10 | 3,97 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 25c Beispiel für Kapselformulierungen**

| Nr. | Formel IX | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,97 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 26a Beispiel für Kapselformulierungen**

| Nr. | Formel X | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 26b Beispiel für Kapselformulierungen**

| Nr. | Formel X | Maisstärke vorver-kleistert | Cross-povidone | Cellulose-Pulver | Maisstärke |
|---|---|---|---|---|---|
| 1 | 0,01 | 2,0 | 20 | 10 | 7,99 |
| 2 | 0,03 | 6,0 | 20 | 10 | 3,79 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 26c Beispiel für Kapselformulierungen**

| Nr. | Formel X | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,97 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 27a Beispiel für Kapselformulierungen**

| Nr. | Formel XI | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 27b Beispiel für Kapselformulierungen**

| Nr. | Formel XI | Maisstärke vorver-kleistert | Cross-povidone | Cellulose-Pulver | Maisstärke |
|---|---|---|---|---|---|
| 1 | 0,01 | 2,0 | 20 | 10 | 7,99 |
| 2 | 0,03 | 6,0 | 20 | 10 | 3,79 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 27c Beispiel für Kapselformulierungen**

| Nr. | Formel XI | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,79 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 28a Beispiel für Kapselformulierungen**

| Nr. | Formel XII | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 28b Beispiel für Kapselformulierungen**

| Nr. | Formel XII | Maisstärke vorver-kleistert | Cross-povidone | Cellulose-Pulver | Maisstärke |
|---|---|---|---|---|---|
| 1 | 0,01 | 2,0 | 20 | 10 | 7,99 |
| 2 | 0,03 | 6,0 | 20 | 10 | 3,79 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 28c Beispiel für Kapselformulierungen**

| Nr. | Formel XII | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,97 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 29a Beispiel für Kapselformulierungen**

| Nr. | Formel XIII | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 29b Beispiel für Kapselformulierungen**

| Nr. | Formel XIII | Maisstärke vorver-kleistert | Cross-povidone | Cellulose-Pulver | Maisstärke |
|---|---|---|---|---|---|
| 1 | 0,01 | 2,0 | 20 | 10 | 7,99 |
| 2 | 0,03 | 6,0 | 20 | 10 | 3,79 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 29c Beispiel für Kapselformulierungen**

| Nr. | Formel XIII | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,97 |
| 3 | 0,05 | 10,0 | 20 | - | 9,9 |
| 4 | 0,1 | 20,0 | 40 | - | 9,9 |
| 5 | 0,3 | 60,0 | 120 | - | - |

**Tabelle 30a Beispiel für Kapselformulierungen**

| Nr. | Formel XIV | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 30b Beispiel für Kapselformulierungen**

| Nr. | Formel XIV | Maisstärke vorver-kleistert | Cross-povidone | Cellulose-Pulver | Maisstärke |
|---|---|---|---|---|---|
| 1 | 0,01 | 2,0 | 20 | 10 | 7,99 |
| 2 | 0,03 | 6,0 | 20 | 10 | 3,79 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 30c Beispiel für Kapselformulierungen**

| Nr. | Formel XIV | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,97 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 31a Beispiel für Kapselformulierungen**

| Nr. | Formel XV | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 31b Beispiel für Kapselformulierungen**

| Nr. | Formel XV | Maisstärke vorver-kleistert | Cross-povidone | Cellulose-Pulver | Maisstärke |
|---|---|---|---|---|---|
| 1 | 0,01 | 2,0 | 20 | 10 | 7,99 |
| 2 | 0,03 | 6,0 | 20 | 10 | 3,79 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 31c Beispiel für Kapselformulierungen**

| Nr. | Formel XV | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,97 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 32a Beispiel für Kapselformulierungen**

| Nr. | Formel XVI | Cross-povidone | Cellulose-Pulver | Maisstärke |
|---|---|---|---|---|
| 1 | 0,01 | 20,0 | 15 | 4,99 |
| 2 | 0,03 | 36,0 | - | 3,97 |
| 3 | 0,05 | 30,0 | - | 9,95 |
| 4 | 0,1 | 20,0 | 10 | 9,9 |
| 5 | 0,3 | 60,0 | - | 9,7 |

**Tabelle 32b Beispiel für Kapselformulierungen**

| Nr. | Formel XVI | Maisstärke vorver-kleistert | Cross-povidone | Cellulose-Pulver | Maisstärke |
|---|---|---|---|---|---|
| 1 | 0,01 | 2,0 | 20 | 10 | 7,99 |
| 2 | 0,03 | 6,0 | 20 | 10 | 3,79 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Tabelle 32c Beispiel für Kapselformulierungen**

| Nr. | Formel XVI | Dextran | Cross-povidone | Cellulose-Pulver | Maisstärke |
|---|---|---|---|---|---|
| 1 | 0,01 | 2,0 | 20 | 10 | 7,99 |
| 2 | 0,3 | 6,0 | 20 | 10 | 3,97 |
| 3 | 0,05 | 10,0 | 20 | - | 9,95 |
| 4 | 0,1 | 20,0 | 40 | - | 9,9 |
| 5 | 0,3 | 60,0 | 120 | - | - |

**Patentansprüche**

1. An vorverkleisterte Starke oder Dextrane gebundene Prostaglandine des E-Types, in denen das C16-Atom substituiert ist.

2. Komplexe von Crosspovidonen, vorverkleisterter Stärke oder Dextranen mit:
(I) 16-Methyl-1-11α 16 RS-Trihydroxyprost-13E-en-9on,

(II) 15-Deoxy-16-hydroxy-17-cyclobutano-$PGE_1$-methylester,
(III) $PGE_1$,
(IV) (17S)-17.20-Dimethyl-trans-$\Delta^2$ $PGE_1$,
(V) 19β-Hydroxy-$PGE_1$,
(VI) (±) (16RS)-15-Deoxy-16-hydroxy-16-methyl-$PGE_1$-methylester,
(VII) 16,16-Dimethyl-trans-$\Delta^2$-$PGE_1$-methylester
(VIII) 2-Decarboxy-2-hydroxy-methyl-$PGE_1$,
(IX) (+)-11α, 16α, β-Dihydroxy-1,9-dioxo-1-(hydroxy-methyl)-16-methyl-13-transprosiene,
(X) 4,5,6-Trinor-3,7-inter-m-phenylen-3-oxa-$PGE_1$,
(XI) (15R)-15-Methyl-$PGE_2$,
(XII) 16,16-Dimethyl-$PGE_2$,
(XIII) 16-Methyl-20-methoxy-$PGE_2$,
(XIV) (±) (16RS)-15-Deoxy-16-hydroxy-16-methyl-$PGE_2$-methylester,
(XV) (+)-4,5-Didehydro-16-phenoxy-ω-tetranor-$PGE_1$-methylester und/oder
(XVI) N-Methan-sulfonyl-10-phenoxy-ω-tetranor-$PGE_2$-amid

3. Komplexe von 16-Methyl-1-11α, 16 RS-Trihydroxyprost-13E-en-9on gemäß Formel (I)

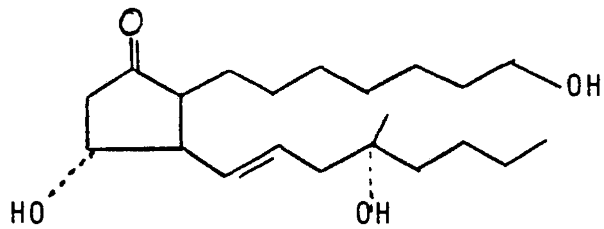

(I)

und Crosspovidonen.

4. Verfahren zur Herstellung stabiler Prostaglandin-Formulierungen, dadurch gekennzeichnet, daß man Prostaglandine des E-Typs, in denen das C16-Atom substituiert ist, in gelöster Form auf Crosspovidone, vorverkleisterte Stärke oder Dextrane aufbringt und anschließend schonend trocknet.

5. Verfahren nach Anspruch 4, dadurch gekennzeichnet, daß man als Prostaglandin des E-Typs 16-Methyl-1-11α, 16 RS-Trihydroxyprost-13E-en-9on gemäß Formel (I) einsetzt.

6. Verfahren nach Anspruch 4 oder 5, dadurch gekennzeichnet, daß man die gelösten Prostaglandine in solcher Menge auf Crosspovidone, vorverkleisterte Stärke oder Dextran aufbringt, daß ein Gewichtsverhältnis von Prostaglandin zu Crosspovidone, vorverkleisterte Stärke oder Dextran zwischen 1 : 1 bis 1 : 10 000 vorliegt.

7. Verfahren nach einem oder mehreren der Ansprüche 4 bis 6, dadurch gekennzeichnet, daß man als schonendes Trocknungsverfahren eine Gefriertrocknung, Vakuumtrocknung, Wirbelschichttrocknung oder Sprühtrocknung einsetzt.

8. Verfahren nach einem oder mehreren der Ansprüche 4 bis 6, dadurch gekennzeichnet, daß die Trocknung so geführt wird, daß der Restwassergehalt des getrockneten Komplexes zwischen 0,1 und 10 Gew.-% beträgt.

9. Verfahren nach einem oder mehreren der Anspruche 4 bis 6, dadurch gekennzeichnet, daß die Prostaglandin-Formulierung nach der Trocknung mit einer Hülle aus Hartgelatine oder Polyethylenglykol überzogen wird.

10. Verwendung von an Crosspovidone, vorverkleisterte Stärke oder Dextrane gebundenen Prostaglandinen des E-Typs, in denen das C16-Atom substituiert ist, zur Herstellung von Arzneimitteln.

11. Formulierung enthaltend an vorverkleisterte Stärke oder Dextran gebundene Prostaglandine des E-Typs, in denen das C16-Atom substituiert ist.

12. Formulierung enthaltend an Crosspovidone, vorverkleisterte Stärke oder Dextran gebundenes 16-Methyl-1-11α, 16 RS-Trihydroxyprost-13E-en-9on der Formel (I).

13. Formulierung enthaltend einen Komplex aus Crosspovidone und 16-Methyl-1-11α, 16 RS-Trihydroxyprost-13E-en-9on der Formel (I).

14. Feste Arzneiform enthaltend an Crosspovidone, vorverkleisterte Stärke oder Dextran gebundene Prostaglandine der Gruppe:
(I) 16-Methyl-1-11α, 16 RS-Trihydroxyprost-13E-en-9on,
(II) 15-Deoxy-16-hydroxy-17-cyclobutano-$PGE_1$-methylester,
(III) $PGE_1$,
(IV) (17S)-17.20-Dimethyl-trans-$\Delta^2$ $PGE_1$,
(V) 19β-Hydroxy-$PGE_1$,
(VI) (±) (16RS)-15-Deoxy-16-hydroxy-16-methyl-$PGE_1$-methylester,
(VII) 16,16-Dimethyl-trans-$\Delta^2$-$PGE_1$-methylester,
(VIII) 2-Decarboxy-2-hydroxy-methyl-$PGE_1$,
(IX) (+)-11α, 16α, β-Dihydroxy-1,9-dioxo-1-(hydroxy-methyl)-16-methyl-13-transprosiene,
(X) 4,5,6-Trinor-3,7-inter-m-phenylen-3-oxa-$PGE_1$,

(XI) (15R)-15-Methyl-$PGE_2$,
(XII) 16,16-Dimethyl-$PGE_2$,
(XIII) 16-Methyl-20-methoxy-$PGE_2$,
(XIV) (±) (16RS)-15-Deoxy-16-hydroxy-16-methyl-$PGE_2$-methylester,
(XV) (+)-4,5-Didehydro-16-phenoxy-ω-tetranor-$PGE_1$-methylester und/oder
(XVI) N-Methan-sulfonyl-10-phenoxy-ω-tetranor-$PGE_2$-amid

15. Tablette enthaltend an Crosspovidone, vorverkleisterte Stärke oder Dextran gebundene Prostaglandine der Gruppe:
(I) 16-Methyl-1-11α, 16 RS-Trihydroxyprost-13E-en-9on,
(II) 15-Deoxy-16-hydroxy-17-cyclobutano-$PGE_1$-methylester,
(III) $PGE_1$,
(IV) (17S)-17.20-Dimethyl-trans-$\Delta^2$ $PGE_1$,
(V) 19β-Hydroxy-$PGE_1$,
(VI) (±) (16RS)-15-Deoxy-16-hydroxy-16-methyl-$PGE_1$-methylester,
(VII) 16,16-Dimethyl-trans-$\Delta^2$-$PGE_1$-methylester,
(VIII) 2-Decarboxy-2-hydroxy-methyl-$PGE_1$,
(IX) (+)-11α, 16α, β-Dihydroxy-1,9-dioxo-1-(hydroxy-methyl)-16-methyl-13-transprosiene,
(X) 4,5,6-Trinor-3,7-inter-m-phenylen-3-oxa-$PGE_1$,
(XI) (15R)-15-Methyl-$PGE_2$,
(XII) 16,16-Dimethyl-$PGE_2$,
(XIII) 16-Methyl-20-methoxy-$PGE_2$,
(XIV) (±) (16RS)-15-Deoxy-16-hydroxy-16-methyl-$PGE_2$-methylester,
(XV) (+)-4,5-Didehydro-16-phenoxy-ω-tetranor-$PGE_1$-methylester und/oder
(XVI) N-Methan-sulfonyl-10-phenoxy-ω-tetranor-$PGE_2$-amid

16. Tablette nach Anspruch 15 enthaltend an Crosspovidone, vorverkleisterte Stärke oder Dextran gebundenes 16-Methyl-1-11α, 16 RS-Trihydroxy-prost-13E-en-9on gemäß der Formel (I).

17. Kapsel enthaltend an Crosspovidone, vorverkleisterte Stärke oder Dextran gebundene Prostaglandine der Gruppe:
(I) 16-Methyl-1-11α, 16 RS-Trihydroxyprost-13E-en-9on,
(II) 15-Deoxy-16-hydroxy-17-cyclobutano-$PGE_1$-methylester,
(III) $PGE_1$,
(IV) (17S)-17.20-Dimethyl-trans-$\Delta^2$ $PGE_2$,
(V) 19β-Hydroxy-$PGE_1$,
(VI) (±) (16RS)-15-Deoxy-16-hydroxy-16-methyl-$PGE_1$-methylester,
(VII) 16,16-Dimethyl-trans-$\Delta^2$-$PGE_1$-methylester,
(VIII) 2-Decarboxy-2-hydroxy-methyl-$PGE_1$,
(IX) (+)-11α, 16α, β-Dihydroxy-1,9-dioxo-1-(hydroxy-methyl)-16-methyl-13-transprosiene,
(X) 4,5,6-Trinor-3,7-inter-m-phenylen-3-oxa-$PGE_1$,
(XI) (15R)-15-Methyl-$PGE_2$,
(XII) 16,16-Dimethyl-$PGE_2$,
(XIII) 16-Methyl-20-methoxy-$PGE_2$,
(XIV) (±) (16RS)-15-Deoxy-16-hydroxy-16-methyl-$PGE_2$-methylester,
(XV) (+)-4,5-Didehydro-16-phenoxy-ω-tetranor-$PGE_1$-methylester und/oder
(XVI) N-Methan-sulfonyl-10-phenoxy-ω-tetranor-$PGE_2$-amid

18. Kapsel nach Anspruch 17 enthaltend an Crosspovidone, vorverkleisterte Stärke oder Dextran gebundenes 16-Methyl-1-11α, 16 RS-Trihydroxyprost-13E-en-9on gemäß der Formel (I).

19. Injektions- und Infusionszubereitungen, enthaltend an Dextran gebundene Prostaglandine des E-Typs, in denen das C16-Atom substituiert ist, bevorzugt 16-Methyl-1-11α, 16 RS-Trihydroxyprost-13E-en-9on der Formel (I).

**Claims**

1. Prostaglandins of the E type, in which the C16 atom is substituted, bound to pre-pasted starch or dextrans.

2. Complexes of crosspovidones, pre-pasted starch or dextrans with:
(I) 16-Methyl-1-11α, 16 RS-trihydroxyprost-13E-en-9-one,
(II) 15-Deoxy-16-hydroxy-17-cyclobutano-$PGE_1$ methyl ester,
(III) $PGE_1$,
(IV) (17S)-17.20-Dimethyl-trans-$\Delta^2$ $PGE_1$,
(V) 19β-Hydroxy-$PGE_1$,
(VI) (±) (16RS)-15-Deoxy-16-hydroxy-16-methyl-$PGE_1$ methyl ester,
(VIL) 16,16-Dimethyl-trans-$\Delta^2$-$PGE_1$ methyl ester,
(VIII) 2-Decarboxy-2-hydroxy-methyl-$PGE_1$,
(IX) (+)-11α, 16α, β-Dihydroxy-1,9-dioxo-1-(hydroxy-methyl)-16-methyl-13-transprosienes,

(X) 4,5,6-Trinor-3,7-inter-m-phenylene-3-oxa-$PGE_1$,
(XI) (15R)-15-Methyl-$PGE_2$,
(XII) 16,16-Dimethyl-$PGE_2$,
(XIII) 16-Methyl-20-methoxy-$PGE_2$,
(XIV) (±) (16RS)-15-Deoxy-16-hydroxy-16-methyl-$PGE_2$ methyl ester,
(XV) (+)-4,5-Didehydro-16-phenoxy-ω-tetranor-$PGE_1$ methyl ester and/or
(XVI) N-Methane-sulphonyl-10-phenoxy-ω-tetranor-$PGE_2$ amide.

3. Complexes of 16-methyl-1-11α, 16 RS-trihydroxyprost-13E-en-9-one according to the formula (I)

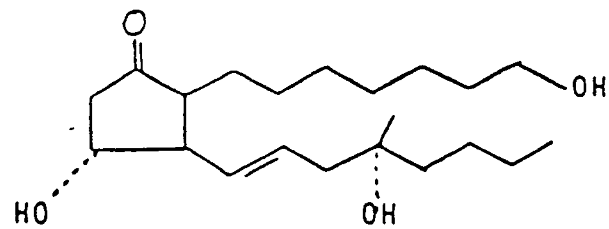

(I)

and crosspovidones.

4. Process for the preparation of stable prostaglandin formulations, characterized in that prostaglandins of the E type, which are substituted on the C16 atom, are applied in dissolved form to crosspovidones, pre-pasted starch or dextrans and are then dried under mild conditions.

5. Process according to Claim 4, characterized in that the prostaglandin of the E type used is 16-methyl-1-11α, 16 RS-trihydroxyprost-13E-en-9-one according to formula (I).

6. Process according to Claim 4 or 5, characterized in that the dissolved prostaglandins are applied to crosspovidones, pre-pasted starch or dextran in an amount such that the weight ratio of prostaglandin to crosspovidones, pre-pasted starch or dextran is between 1 : 1 and 1 : 10,000.

7. Process according to one or more of Claims 4 to 6, characterized in that freeze-drying, vacuum drying, fluidized bed drying or spray drying is used as mild drying process.

8. Process according to one or more of Claims 4 to 6, characterized in that drying is carried out in a manner such that the residual water content of the dried complex is between 0.1 and 10 % by weight.

9. Process according to one or more of Claims 4 to 6, characterized in that the prostaglandin formulation is, after drying, coated with a covering of hard gelatin or polyethylene glycol.

10. Use of prostaglandins of the E type, in which the C16 atom is substituted, which are bound to crosspovidones, pre-pasted starch or dextrans, for the preparation of medicaments.

11. Formulation containing prostaglandins of the E type, in which the C16 atom is substituted, which are bound to pre-pasted starch or dextran.

12. Formulation containing 16-methyl-1-11α, 16 RS-trihydroxyprost-13E-en-9-one of the formula (I) bound to crosspovidones, pre-pasted starch or dextran.

13. Formulation containing a complex of crosspovidones and 16-methyl-1-11α, 16 RS-trihydroxyprost-13E-en-9-one of the formula (I).

14. Solid drug form containing prostaglandins, bound to crosspovidones, pre-pasted starch or dextran, of the group:
(I) 16-Methyl-1-11α, 16 RS-trihydroxyprost-13E-en-9-one,
(II) 15-Deoxy-16-hydroxy-17-cyclobutano-$PGE_1$ methyl ester,
(III) $PGE_1$,
(IV) (17S)-17.20-Dimethyl-trans-$\Delta^2$ $PGE_1$,
(V) 19β-±-Hydroxy-$PGE_1$,
(VI) (±) (16RS)-15-Deoxy-16-hydroxy-16-methyl-$PGE_1$ methyl ester,
(VII) 16,16-Dimethyl-trans-$\Delta^2$-$PGE_1$ methyl ester,
(VIII) 2-Decarboxy-2-hydroxy-methyl-$PGE_1$,
(IX) (+)-11α, 16α, β-Dihydroxy-1,9-dioxo-1-(hydroxy-methyl)-16-methyl-13-transprosienes,
(X) 4,5,6-Trinor-3,7-inter-m-phenylene-3-oxa-$PGE_1$,
(XI) (15R)-15-Methyl-$PGE_2$,
(XII) 16,16-Dimethyl-$PGE_2$,
(XIII) 16-Methyl-20-methoxy-$PGE_2$,
(XIV) (±) (16RS)-15-Deoxy-16-hydroxy-16-methyl-$PGE_2$ methyl ester,
(XV) (+)-4,5-Didehydro-16-phenoxy-ω-tetranor-$PGE_1$ methyl ester and/or
(XVI) N-Methane-sulphonyl-10-phenoxy-ω-tetranor-$PGE_2$-amide.

15. Tablet containing prostaglandins, bound to crosspovidones, pre-pasted starch or dextran, of the group:
(I) 16-Methyl-1-11α, 16 RS-trihydroxyprost-13E-en-9-one,
(II) 15-Deoxy-16-hydroxy-17-cyclobutano-$PGE_1$ methyl ester,
(III) $PGE_1$,

(IV) (17S)-17.20-Dimethyl-trans-$\Delta^2$ $PGE_1$,
(V) 19β-Hydroxy-$PGE_1$,
(VI) (±) (16RS)-15-Deoxy-16-hydroxy-16-methyl-$PGE_1$ methyl ester,
(VII) 16,16-Dimethyl-trans-$\Delta^2$-$PGE_2$ methyl ester,
(VIII) 2-Decarboxy-2-hydroxy-methyl-$PGE_1$,
(IX) (+)-11α, 16α, β-Dihydroxy-1,9-dioxo-1-(hydroxy-methyl)-16-methyl-13-transprosienes,
(X) 4,5,6-Trinor-3,7-inter-m-phenylene-3-oxa-$PGE_1$,
(XI) (15R)-15-Methyl-$PGE_2$,
(XII) 16,16-Dimethyl-$PGE_2$,
(XIII) 16-Methyl-20-methoxy-$PGE_2$,
(XIV) (±) (16RS)-15-Deoxy-16-hydroxy-16-methyl-$PGE_2$ methyl ester,
(XV) (+)-4,5-Didehydro-16-phenoxy-ω-tetranor-$PGE_1$ methyl ester and/or
(XVI) N-Methane-sulphonyl-10-phenoxy-ω-tetranor-$PGE_2$ amide.

16. Tablet according to Claim 15, containing 16-methyl-1-11α, 16 RS-trihydroxyprost-13E-en-9-one according to the formula (I) bound to crosspovidones, pre-pasted starch or dextran.

17. Capsule containing prostaglandins, bound to crosspovidones, pre-pasted starch or dextran, of the group:
(I) 16-Methyl-1-11α, 16 RS-trihydroxyprost-13E-en-9-one,
(II) 15-Deoxy-16-hydroxy-17-cyclobutano-$PGE_1$ methyl ester,
(III) $PGE_1$,
(IV) (17S)-17.20-Dimethyl-trans-$\Delta^2$ $PGE_1$
(V) 19β-Hydroxy-$PGE_1$,
(VI) (±) (16RS)-15-Deoxy-16-hydroxy-16-methyl-$PGE_1$ methyl ester,
(VII) 16,16-Dimethyl-trans-$\Delta^2$-$PGE_1$ methyl ester,
(VIII) 2-Decarboxy-2-hydroxy-methyl-$PGE_1$,
(IX) (+)-11α, 16α, β-Dihydroxy-1,9-dioxo-1-(hydroxy-methyl)-16-methyl-13-transprosienes,
(X) 4,5,6-Trinor-3,7-inter-m-phenylene-3-oxa-$PGE_1$,
(XI) (15R)-15-Methyl-$PGE_2$,
(XII) 16,16-Dimethyl-$PGE_2$,
(XIII) 16-Methyl-20-methoxy-$PGE_2$,
(XIV) (±) (16RS)-15-Deoxy-16-hydroxy-16-methyl-$PGE_2$ methyl ester,
(XV) (+)-4,5-Didehydro-16-phenoxy-ω-tetranor-$PGE_1$ methyl ester and/or
(XVI) N-Methane-sulphonyl-10-phenoxy-ω-tetranor-$PGE_2$ amide.

18. Capsule according to Claim 17, containing 16-methyl-1-11α, 16 RS-trihydroxyprost-13E-en-9-one according to the formula (I) bound to crosspovidones, pre-pasted starch or dextran.

19. Injection and infusion preparations containing prostaglandins of the E type, in which the C16 atom is substituted, preferably 16-methyl-1-11α, 16 RS-trihydroxyprost-13E-en-9-one of the formula (I), which are bound to dextran.

**Revendications**

1. Prostaglandines de type E liées à des dextranes ou de l'amidon prégélatinisé, dans lesquelles l'atome de carbone occupant la position 16 est substitué.

2. Complexes de Crosspovidones, d'amidon prégélatinisé ou de dextranes avec:
(I) la 16-méthyl-1-11α, 16 RS-trihydroxyprost-13 E-én-9-one,
(II) l'ester méthylique de la 15-déoxy-16-hydroxy-17-cyclobutano-$PGE_1$,
(III) la $PGE_1$,
(IV) la (17S)-17.20-diméthyl-trans-$\Delta^2$ $PGE_1$,
(V) la 19β-hydroxy-$PGE_1$,
(VI) l'ester méthylique de (±) (16RS)-15-déoxy-16-hydroxy-16-méthyl-$PGE_1$,
(VII) l'ester méthylique de la 16,16-diméthyl-trans-$\Delta^2$-$PGE_1$,
(VIII) la 2-décarboxy-2-hydroxy-méthyl-$PGE_1$,
(IX) le (+)-11α, 16α, β-dihydroxy-1,9-dioxo-1-(hydroxyméthyl)-16-méthyl-13-transprosiène,
(X) la 4,5,6-trinor-3,7-inter-m-phénylène-3-oxa-$PGE_1$,
(XI) la (15R)-15-méthyl-$PGE_2$,
(XII) la 16,16-diméthyl-$PGE_2$,
(XIII) la 16-méthyl-20-méthoxy-$PGE_2$,
(XIV) l'ester méthylique de la (±) (16RS)-15-déoxy-16-hydroxy-16-méthyl-$PGE_2$,
(XV) l'ester méthylique de la (+)-4,5-didéhydro-16-phénoxy-ω-tétranor-$PGE_1$ et/ou
(XVI) l'amide de la N-méthane-sulfonyl-10-phénoxy-ω-tétranor-$PGE_2$.

3. Complexes de la 16-méthyl-1-11α, 16 RS-trihydroxyprost-13E-én-9-one répondant à la formule (I):

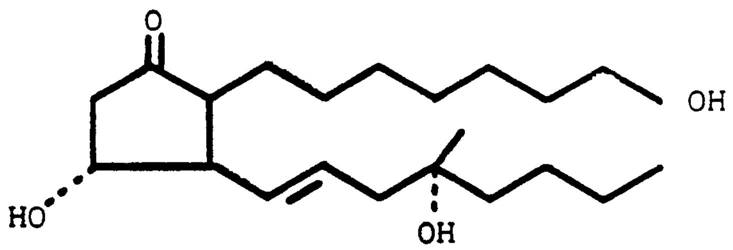

(I)

et de Crosspovidones.

4. Procédé de préparation de formulations stables de prostaglandines, caractérisé en ce qu'on applique des prostaglandines de type E dans lesquelles l'atome de carbone occupant la position 16 est substitué, sous forme dissoute, sur de la Crosspovidone, de l'amidon prégélatinisé ou des dextranes, puis on sèche avec ménagement.

5. Procédé selon la revendication 4, caractérisé en ce que, comme prostaglandine de type E, on utilise la 16-méthyl-1-11α, 16 RS-trihydroxyprost-13E-én-9-one de formule (I).

6. Procédé selon la revendication 4 ou 5, caractérisé en ce qu'on applique les prostaglandines dissoutes sur la Crosspovidone, l'amidon prégélatinisé ou le dextrane en une quantité calculée de telle sorte que l'on obtienne un rapport pondéral se situant entre 1 : 1 et 1 : 10.000 entre la prostaglandine et la Crosspovidone, l'amidon prégélatinisé ou le dextrane.

7. Procédé selon une ou plusieurs des revendications 4 à 6, caractérisé en ce que, pour procéder à un séchage avec ménagement, on recourt à une lyophilisation, à un séchage sous vide, à un séchage en lit fluidisé ou à un séchage par pulvérisation.

8. Procédé selon une ou plusieurs des revendications 4 à 6, caractérisé en ce qu'on effectue le séchage de telle sorte que la teneur résiduelle en eau du complexe séché se situe entre 0,1 et 10 % en poids.

9. Procédé selon une ou plusieurs des revendications 4 à 6, caractérisé en ce que, après le séchage, la formulation de prostaglandine est revêtue d'une enveloppe en gélatine dure ou en polyéthylène-glycol.

10. Utilisation de prostaglandines de type E liées à des Crosspovidones, à de l'amidon prégélatinisé ou à des dextranes et dans lesquelles l'atome de carbone occupant la position 16 est substitué, pour la préparation de médicaments.

11. Formulation contenant des prostaglandines de type E liées à de l'amidon prégélatinisé ou à un dextrane et dans lesquelles l'atome de carbone occupant la position 16 est substitué.

12. Formulation contenant la 16-méthyl-1-11α, 16 RS-trihydroxyprost-13E-én-9-one de formule (I) liée à une Crosspovidone, à de l'amidon prégélatinisé ou à un dextrane.

13. Formulation contenant un complexe de Crosspovidone et de 16-méthyl-1-11α, 16 RS-trihydroxyprost-13E-én-9-one de formule (I).

14. Forme médicamenteuse solide contenant des prostaglandines liées à une Crosspovidone, à de l'amidon prégélatinisé ou à un dextrane et choisies parmi le groupe comprenant:
(I) la 16-méthyl-1-11α, 16 RS-trihydroxyprost-13E-én-9-one,
(II) l'ester méthylique de la 15-déoxy-16-hydroxy-17-cyclobutano-$PGE_1$,
(III) la $PGE_1$,
(IV) la (17S)-17.20-diméthyl-trans-$\Delta^2$ $PGE_1$,
(V) la 19β-hydroxy-$PGE_1$,
(VI) l'ester méthylique de la (±) (16RS)-15-déoxy-16-hydroxy-16-méthyl-$PGE_1$,
(VII) l'ester méthylique de la 16,16-diméthyl-trans-$\Delta^2$-$PGE_1$,
(VIII) la 2-décarboxy-2-hydroxy-méthyl-$PGE_1$,
(IX) le (+)-11α, 16α, β-dihydroxy-1,9-dioxo-1-(hydroxyméthyl)-16-méthyl-13-transprosiène,
(X) la 4,5,6-trinor-3,7-inter-m-phénylène-3-oxa-$PGE_1$,
(XI) la (15)-15-méthyl-$PGE_2$,
(XII) la 16,16-diméthyl-$PGE_2$,
(XIII) la 16-méthyl-20-méthoxy-$PGE_2$,
(XIV) l'ester méthylique de la (±) (16RS)-15-déoxy-16-hydroxy-16-méthyl-$PGE_2$,
(XV) l'ester méthylique de la (+)-4,5-didéhydro-16-phénoxy-ω-tétranor-$PGE_1$ et/ou
(XVI) l'amide de la N-méthane-sulfonyl-10-phénoxy-ω-tétranor-$PGE_2$.

15. Comprimés contenant des prostaglandines liées à une Crosspovidone, à de l'amidon prégélatinisé ou à un dextrane et choisies parmi le groupe:
(I) la 16-méthyl-1-11α, 16 RS-trihydroxyprost-13E-én-9-one,
(II) l'ester méthylique de la 15-déoxy-16-hydroxy-17-cyclobutano-$PGE_1$,
(III) la $PGE_1$,
(IV) la (17S)-17.20-diméthyl-trans-$\Delta^2$ $PGE_1$,
(V) la 19β-hydroxy-$PGE_1$,
(VI) l'ester méthylique de la (±) (16RS)-15-déoxy-16-hydroxy-16-méthyl-$PGE_1$,

(VII) l'ester méthylique de la 16,16-diméthyl-trans-$\Delta^2$-$PGE_1$,
(VIII) la 2-décarboxy-2-hydroxy-méthyl-$PGE_1$,
(IX) le (+)-11α, 16α, β-dihydroxy-1,9-dioxo-1-(hydroxy-méthyl)-16-méthyl-13-transprosiène,
(X) la 4,5,6-trinor-3,7-inter-m-phénylène-3-oxa-$PGE_1$,
(XI) la (15R)-15-méthyl-$PGE_2$,
(XII) la 16,16-diméthyl-$PGE_2$,
(XIII) la 16-méthyl-20-méthoxy-$PGE_2$,
(XIV) l'ester méthylique de la (±) (16RS)-15-déoxy-16-hydroxy-16-méthyl-$PGE_2$,
(XV) l'ester méthylique de la (+)-4,5-didéhydro-16-phénoxy-ω-tétranor-$PGE_1$ et/ou
(XVI) l'amide de la N-méthane-sulfonyl-10-phénoxy-ω-tétranor-$PGE_2$.

16. Comprimés selon la revendication 15 contenant la 16-méthyl-1-11α, 16 RS-trihydroxyprost-13E-én-9-one de formule (I) liée à une Crosspovidone, à de l'amidon prégélatinisé ou à un dextrane.

17. Capsule contenant des prostaglandines liées à une Crosspovidone, à de l'amidon prégélatinisé ou à un dextrane et choisies parmi le groupe comprenant:
(I) la 16-méthyl-1-11α, 16 RS-trihydroxyprost-13E-én-9-one,
(II) l'ester méthylique de la 15-déoxy-16-hydroxy-17-cyclobutano-$PGE_1$,
(III) la $PGE_1$,
(IV) la (17S)-17.20-diméthyl-trans-$\Delta^2$ $PGE_1$,
(V) la 19β-hydroxy-$PGE_1$,
(VI) l'ester méthylique de la (±) (16RS)-15-déoxy-16-hydroxy-16-méthyl-$PGE_1$,
(VII) l'ester méthylique de la 16,16-diméthyl-trans-$\Delta^2$-$PGE_1$,
(VIII) la 2-décarboxy-2-hydroxy-méthyl-$PGE_1$,
(IX) le (+)-11α, 16α, β-dihydroxy-1,9-dioxo-1-(hydroxy-méthyl)-16-méthyl-13-transprosiène,
(X) la 4,5,6-trinor-3,7-inter-m-phénylène-3-oxa-$PGE_1$,
(XI) la (15R)-15-méthyl-$PGE_2$,
(XII) la 16,16-diméthyl-$PGE_2$,
(XIII) la 16-méthyl-20-méthoxy-$PGE_2$,
(XIV) l'ester méthylique de la (±) (16RS)-15-déoxy-16-hydroxy-16-méthyl-$PGE_2$,
(XV) l'ester méthylique de la (+)-4,5-didéhydro-16-phénoxy-ω-tétranor-$PGE_1$, et/ou
(XVI) l'amide de la N-méthane-sulfonyl-10-phénoxy-ω-tétranor-$PGE_2$.

18. Capsule selon la revendication 17 contenant la 16-méthyl-1-11α, 16 RS-trihydroxyprost-13E-én-9-one de formule (I) liée à une Crosspovidone, à de l'amidon prégélatinisé ou à un dextrane.

19. Préparations pour injections et infusions contenant des prostaglandines de type E liées à un dextrane et dans lesquelles l'atome de carbone occupant la position 16 est substitué, de préférence, la 16-méthyl-1-11α, 16 RS-trihydroxyprost-13E-én-9-one de formule (I).